# EUROPEAN PATENT SPECIFICATION

(11) **EP 1 150 570 B1**
(45) Date of publication and mention of the grant of the patent: **12.07.2006**
(21) Application number: 00909957.3
(22) Date of filing: 21.01.2000
(51) Int. Cl.: C12N 5/06, A61K 35/12, A61K 35/14, C12N 5/08, A61K 39/00, A61K 48/00, A61P 37/02, A61K 38/18, A61K 38/34

(54) **ACTIVATION OF REGULATORY T CELLS BY ALPHA-MELANOCYTE STIMULATING HORMONE**
AKTIVIERUNG VON REGULATORISCHEN T ZELLEN DURCH EIN ALPHA-MELANOCYTEN STIMULIERENDES HORMON
ACTIVATION DE LYMPHOCYTES T REGULATEURS PAR UNE HORMONE STIMULANT DES ALPHA-MELANOCYTES

(30) Priority: 22.01.1999 US 116851 P; 30.09.1999 US 156788 P
(43) Date of publication of application: 07.11.2001
(73) Proprietor: THE SCHEPENS EYE RESEARCH INSTITUTE, INC., Boston, MA 02114 (US)
(72) Inventor: TAYLOR, Andrew, W., Waltham, MA 02451 (US); NISHIDA, Tomomi, Kanazawa Yokohama Kanagawa 236-0054 (JP)
(74) Representative: Schurack, Eduard F.
(86) International application number: PCT/US2000/001608
(87) International publication number: WO 2000/042856

(56) References cited:
- TAYLOR A W ET AL: "Alpha-melanocyte-stimulating hormone suppresses antigen-stimulated T cell production of gamma-interferon." NEUROIMMUNOMODULATION, vol. 1, no. 3, 1994, pages 188-194, XP008005225 ISSN: 1021-7401
- TAYLOR A W & STREILEN J W: "Alpha-melanocyte stimulating hormone prevents induction of effector T-cell inflammatory activity." FASEB JOURNAL, vol. 10, no. 6, 1996, page A1206 XP002927877 Joint Meeting of the American Society for Biochemistry and Molecular Biology, the American Society for Investigative Pathology and the American Association of Immunologists; New Orleans, Louisiana, USA; 2-6 June 1996 ISSN: 0892-6638
- LIPTON J M & CATANIA A: "Anti-inflammatory actions of the neuroimmunomodulator alpha-MSH." IMMUNOLOGY TODAY, vol. 18, no. 3, 1997, pages 140-145, XP002902200 ISSN: 0167-5699
- TAYLOR A W: "Th3 cells are induced by alpha-melanocyte stimulating hormone (alpha-MSH)." FASEB JOURNAL, vol. 13, no. 4 PART 1, 12 March 1999 (1999-03-12), page A607 XP002203897 Annual Meeting of the Professional Research Scientists for Experimental Biology 99; Washington D.C., USA; 17-21 April 1999 ISSN: 0892-6638
- TAYLOR A W & NAMBA K: "In vitro induction of CD25+ CD4+ regulatory T cells by the neuropeptide alpha-melanocyte stimulating hormone (alpha-MSH)." IMMUNOLOGY AND CELL BIOLOGY, vol. 79, no. 4, August 2001 (2001-08), pages 358-367, XP002203898 ISSN: 0818-9641
- TAYLOR A.W. ET AL.: 'Alpha-Melanocyte Stimulating Hormone Inhibits IFN-Gamma-Dependent T-Cell and Macrophage Participation in Immunogenic Inflammation' J. IMMUNOL., vol. 150, no. 8, 15 April 1993, page 129A, ABSTRACT 726, XP002927873
- DUVAUX-MIRET O. ET AL.: 'Immunosuppression in the Definitive and Intermediate Hosts of the Human Parasite Schistosoma Mansoni by Release of Immunoactive Neuropeptides' PROC. NATL. ACAD. SCI. USA, vol. 89, January 1992, pages 778 - 781, XP002927874
- SAPERSTEIN A. ET AL.: 'Interleukin 1beta Mediates Stress-Induced Immunosuppression Via Corticotropin-Releasing Factor' ENDOCRINOLOGY, vol. 130, no. 1, January 1992, pages 152 - 158, XP002927875
- RHEINS L.A. ET AL.: 'Alpha-Melanocyte Stimulating Hormone Modulates Contact Hypersensitivity Responsiveness in C57/BL6 Mice' J. INVEST. DERM., vol. 93, no. 4, October 1989, pages 511 - 517, XP002927876
- TAYLOR A.W. ET AL.: 'Alpha-Melanocyte Stimulating Hormone Prevents Induction of Effector T-Cell Inflammatory Activity' FASEB J., vol. 10, no. 6, 30 April 1996, page A1206, ABSTRACT 1194, XP002927877
- TAYLOR ET AL: 'Identification of alpha-melanocyte stimulating hormone as a potential immunosuppressive factor in aqeuious humor' CURRENT EYE RESEARCH vol. 11, no. 12, 1992, pages 1199 - 1206
- CONSTANT ET AL: 'Induction of TH1 and TH2 CD4+ T cell responses: The alternative approaches' ANN. REV. IMMUNOL. vol. 15, 1997, pages 297 - 322

## Description

### FIELD OF THE INVENTION

The present invention relates to the regulation of T cell-mediated inflammation.

### BACKGROUND OF THE INVENTION

The induction of a delayed type hypersensitivity (DTH) response is dependent on activation of IFN-γ producing Th1 cells (1). The activation not only requires cognate antigen presenting cells, but also a microenvironment that favors activation and development of the DTH-mediating Th1 cells. To regulate activation of a DTH response, several mechanisms are physiologically employed, such as apoptosis and anergy (2). In addition, soluble factors in the regional microenvironment of T cell activation can also influence the course of a T cell response (3). The presence of specific cytokines can favor expression of specific effector responses while suppressing others (4-8). Previously we have found that in the presence of the neuropeptide α-melanocyte stimulating hormone (α-MSH), antigen-activated Th1 cells were suppressed in their IFN-γ production but continued to proliferate, suggesting that α-MSH may regulate selective effector T cell activities (9).

The neuropeptide α-MSH is an evolutionarily conserved tridecapeptide derived from the endoproteolytic cleavage of adrenocorticotropic hormone (ACTH) which is in turn a post-translational product of pro-opiomelanocortin hormone (POMC) (10). Initially, α-MSH was described as a pituitary hormone that mediates melanogenises in amphibians (11). In mammals, α-MSH is able to mediate melanogenises and neurotransmitter activities; however, α-MSH is most potent in functioning as a neuroimmunomodulator (12). α-MSH suppresses inflammation mediated by host defense mechanisms of innate (endotoxin mediated) and of adaptive immunity (T cell mediated). Its anti-inflammatory activity has suggested that α-MSH functions as a necessary physiological regulator of inflammation.

One of the first indications of a link between the immune and nervous systems was in the induction of fever mediated by the systemic effects of IL-1, TNF or endotoxin (Reviewed in (12)). Intracerebroventricular (*icv*) injection of α-MSH suppresses endotoxin and inflammatory cytokine induced fever. Peripheral injections of α-MSH, although at much higher concentrations then central injections, were also effective in suppressing fever. Injections of anti-α-MSH antibodies icv to neutralize CNS α-MSH activity enhanced IL-1 induced febrile response (13). In addition, during acute phase responses α-MSH concentration in plasma and in discrete sites of the CNS are elevated (14, 15). These findings demonstrate that α-MSH antagonizes the CNS response to IL-1, TNF, and endotoxin to regulate the intensity of the febrile response.

Localized peripheral inflammatory responses induced by IL-1, TNF, and endotoxin are also suppressed by α-MSH, regardless if α-MSH is delivered via icv, intravascular, or direct injection to the site (16). Endotoxin and interferon (IFN)-γ activated macrophages cultured with α-MSH are suppressed in generating nitric oxide and in producing TNF and chemokines (17, 18). Also, α-MSH suppresses *in vivo* neutrophil migration in response to endotoxin (18, 19). These findings further indicate that α-MSH antagonizes the activity of inflammatory cytokines and also their synthesis. In addition, α-MSH induces its own production and expression of its receptors on macrophages (17), suggesting that α-MSH can regulate an inflammatory response through autocrine mechanisms. Therefore, various inflammatory mediating events could trigger production of α-MSH that in turn regulates the extent of the inflammatory response. Intravenous injection of α-MSH at the time of applying skin-reactive chemicals suppresses systemic induction and regional expression of contact hypersensitivity (20) leading to hapten-specific tolerance (21). It has recently been found that α-MSH at physiological concentrations induces IL-10 production by antigen presenting cells (22). It is possible that α-MSH indirectly promotes tolerance to hapten by inducing IL-10 production by hapten-presenting APC. Therefore, a regulatory cytokine network initiated by α-MSH could suppress both induction and expression of contact hypersensitivity. However, such a regulatory network does not preclude the possibility that α-MSH can directly suppress or affect the responding T cells. That possibility is the focus of the present invention. Suppression of delayed type hypersensitivity (DTH) is mediated in part by α-MSH within the immune privileged ocular microenvironment (9, 23). The fluid filling the anterior chamber of mammalian eyes, aqueous humor, contains bioactive α-MSH (23). When α-MSH activity was neutralized, aqueous humor was unable to suppress Th1 activity by activated primed T cells *in vitro* (9). Cultures of Th1 cells stimulated with antigen and antigen presenting cells are suppressed in their IFN-γ production by α-MSH, while their proliferation is unaffected (9). This observation suggests that the signals needed by T cells for IFN-γ production are inhibited by α-MSH, whereas the signals for proliferation are not suppressed. Since both the antigen presenting cells and T cells are affected by α-MSH, when only the primed Th1 cells are pretreated with α-MSH, the production of IFN-γ is profoundly suppressed. However, T cell proliferation again is unaffected (9). Therefore, these results suggest that T cells are receptive to α-MSH, and are suppressed in mediating inflammatory activity when activated in the presence of α-MSH. The present invention results from characterizing the effects of α-MSH on TCR-stimulated primed T cells, which has shown that the T cells are target cells for α-MSH regulation, and that its suppression of IFN-γ production is due to α-MSH deflecting Th1 cells away from their expected inflammatory response toward a suppressive response.

Over the past 30 years, research into the mechanisms of ocular immune privilege has lead to the understanding that it is an active process mediated in part by the constitutive production of immunosuppressive factors within the ocular microenvironment.^{A1-A3} Immune privilege involves mechanisms that suppress induction of an inflammatory immune response within the eye. As mentioned, this suppression of the induction of immunity to antigen in the eye is mediated in part by immunosuppressive factors found in aqueous humor.^{A3-A7} The constitutive expression of these immunosuppressive factors appears to regulate systemic and regional immune responses to antigen within the ocular microenvironment.

The activation, type, and intensity of an effector T cell response is not limited to antigen sensitivity alone, but also to local immunoregulatory mechanisms to which neurologically derived factors, such as α-MSH, can contribute. This regional regulation insures that the most effective immune defense is mounted in proportion with preserving the unique functionality of the affected tissue. As mentioned, an extreme example of regional immunity is the immune-privileged microenvironment of the ocular anterior chamber.^{B1} Within this microenvironment, delayed type hypersensitivity-mediating T cells are suppressed.^{A9} This suppression is mediated by factors constitutively produced within the anterior chamber.^{B4-B8} Specific neuropeptides are present within the ocular microenvironment that help to maintain the immunosuppression.^{A1} Changes in neuropeptide expression by neurons that innervate ocular tissues are associated with loss of immune privilege.^{B10}

The mediators of the immunosuppression in eyes are found in the aqueous humor, as first demonstrated by Kaiser et al., who suppressed various *in vitro* T cell assays with normal aqueous humor, and by Streilein and Cousins, who showed that when T cells primed for Th1 activity were pretreated with aqueous humor, they failed to mediate the expected inflammatory response in a local adoptive transfer of DTH assay in skin.^{A2, A3} More recently, it has been reported that primed T cells activated in the presence of aqueous humor were deflected from an expected Th1 response to a Th3 response.^{A8} Such aqueous humor-induced T cells produced TGF-β, suggesting a Th3 phenotype, and suppressed IFN-γ production by other, Th1 type cells. However, it was not shown, prior to the present work, whether these aqueous humor-induced regulatory T cells can suppress DTH.

Several factors in aqueous humor have the potential to influence effector T cell activities.^{A1} Of the many factors in aqueous humor, the present experiments examine the ability of two factors, alpha-melanocyte stimulating hormone (α-MSH) and transforming growth factor-β2 (TGF-β2), to induce regulatory T cells.^{A8-A11} In the presence of α-MSH, activated primed T cells proliferate but are suppressed in their IFN-γ production. ^{A10} This effect of α-MSH, which is independent of IL-4, has suggested that α-MSH mediates differential responses in T cells to TCR-stimulation. Recently it has been found that TGF-β mediates its own production by T cells ^{A11}. These past findings failed to show whether or not these immunosuppressive factors could have a role in the apparent induction of regulatory T cells by aqueous humor. The findings presented here definitively show that α-MSH alone, or α-MSH in conjunction with TGF-β2, mediate(s) induction of TGF-β-producing, regulatory T cells that suppress DTH and may be Th3 cells. In addition to the direct immunosuppressive effects of these aqueous humor factors, the regulatory T cells they induce may also contribute to the normal immunosuppressive microenvironment of the eye by the cells' TGF-β production and suppression of Th1 cell activity. The results here suggest that within the normal ocular microenvironment, there is a potential for Th3 cell induction that supports the immunosuppressive microenvironment of the eye and that possibly mediates peripheral tolerance to ocular autoantigens.

More specifically, α-MSH, which occurs in aqueous humor, has recently been found to suppress IFN-γ production by, but not proliferation by, activated effector T cells.^{B11} This suggests that α-MSH may regulate regional induction of specific effector T cell responses. The thirteen-amino acid long α-MSH (1.6 kDa MW) is encoded within the pro-opiomelanocortin hormone (POMC) gene, and is released from the POMC protein through two endoproteolytic cleavage steps.^{B12, B13} It has a fundamental role in modulating innate host defense mechanisms in mammals, which contrasts to its original description as an amphibian melanin-inducing factor.^{B14,B15} Systemic and central injections of α-MSH suppress innate inflammatory responses induced by endotoxin, IL-1 and TNF (as opposed to adaptive, T cell-mediated immunity). Thus, α-MSH suppresses macrophage-reactive oxygen intermediates and nitric oxide generation, as well as production of inflammatory cytokines.^{B16-B21} In addition, α-MSH induces its own production and receptor expression on the macrophages promoting autocrine suppression of inflammatory-macrophage activities. Also, α-MSH suppresses macrophage and neutrophil chemotactic responses to chemokines and microbial chemoattractants.^{B19, B22} Macrophages, keratinocytes, centrally derived neurons, and possibly any cell that can synthesize POMC, are sources of α-MSH.^{B21, B23, B24} Normal mammalian aqueous humor (the fluid filling the ocular anterior chamber) constitutively expresses, on average, 30 pg/ml of α-MSH. ^{B7}

Much is known about various immunomodulatory effects of α-MSH and of aqueous humor, which contains not only α-MSH but also TGF-β2 and many other factors. However, prior to the present work, much remained unknown about the role of α-MSH and TGF-β2 in regulating T -cell mediated inflammation through T cell networks.

### BRIEF SUMMARY OF THE INVENTION

The present work shows that α-MSH mediates the induction of TGF-β-producing, CD4+/CD25+, regulatory T cells that suppress the activation of other, effector T cells. Thus, α-MSH suppresses T cell-mediated inflammation and mediates selective production of T cell lymphokines. TGF-β2 enhances α-MSH mediated induction of regulatory T cells while TGF-β1 suppresses that induction.

The present invention relates to the discovery that treatment of primed T cells (also called memory or armed T cells) with certain immunomodulating factors, alpha-Melanocyte Stimulating Hormone (α-MSH) in conjunction with Transforming Growth Factor-β2 (TGF-β2), activates regulatory T cells that express both the T helper marker, CD4, and the T cell activation marker, CD25, and produce TGF-β (suggesting a Th3 phenotype). These regulatory T cells suppress activation of other inflammation-mediating T cells (primarily of the Th1 type). Such α-MSH treatment has induced *in vitro* activation of regulatory T cells specific to a particular antigen, e.g., an ocular autoantigen. These α-MSH-induced regulatory T cells were injected into mice at the same time that they were being immunized to induce experimental autoimmune uveitis (EAU), or when the mice were about to have symptoms of EAU. In both cases, EAU in those mice was suppressed - the mice showed no symptoms of autoimmune disease.

Therefore, the present invention provides a treatment for any autoimmune disorder or disease. A combination of α-MSH and TGF-β2 may be used, under certain conditions, to generate regulatory T cells against any autoantigen implicated in an autoimmune disease. Induction of antigen-specific regulatory T cells by α-MSH may also be used to prevent transplant graft rejection. Since the regulatory T cells activated by α-MSH are antigen-specific, they can also be generated against transplant antigens that are targeted by the immune system during transplant rejection.

Regulation of T cell activity is needed for maintaining tolerance to autoantigens. One of the regulatory mechanisms is mediated by factors produced by cells within a localized tissue microenvironment. One of these regulating factors is the neuropeptide, α-melanocyte stimulating hormone (α-MSH), which suppresses immunogenic inflammation. Th1 cells are suppressed from mediating inflammation when they are activated in the presence of α-MSH. Although there is proliferation, α-MSH suppresses IFN-γ production and possibly the secretion of IL-4 by T cell receptor (TCR)-stimulated, primed T cells. Such α-MSH treated T cells produce enhanced levels of TGF-β2. These TGF-β2-producing T cells have Th3 cells characteristics and suppress IFN-γ production by other activated Th1 cells. Therefore, α-MSH suppression of Th1 cell activity is a result of α-MSH deviating the Th1 response into a regulatory T response, i.e., a Th3 response. The presence of α-MSH enhanced tyrosine phosphorylation of CD3ζ chains, but not of CD3ε chains. Hence, α-MSH appears to mediate immune deviation through induction of differential TCR-associated signals in T cells. The ability of α-MSH to mediate induction of regulatory Th3 cells implies that this neuropeptide has an important systemic and regional role in mediating and maintaining peripheral tolerance, especially in such tissues as the eye and the brain, which contain constitutive levels of α-MSH.

Even though the regulatory T cells are activated by α-MSH in an antigen-specific manner, their action is nonspecific and general to the site of their activation. That is, a regulatory T cell is activated by the presence of a specific antigen to which that T cell has been primed, and by the presence of α-MSH, but once activated, it releases factors that are immunosuppressive generally, i.e., factors that suppress the inflammatory activities of other, effector T cells, primarily Th1 cells.

Therefore, the immunosuppressive or immunoregulatory medicament of the invention does not require generating regulatory T cells to all the tissue antigens involved in the autoimmune disorder or the transplantation being regulated. The regulatory T cell induction procedure can be standardized to a specific antigen that is injected into the autoimmune-diseased site or transplant site. Any accessible tissue site that may suffer from damaging immune responses, can be treated with the medicaments of the invention, without having to know the exact antigen triggering the immune response causing the disease or graft rejection. In many cases of autoimmune disease, there is no clear characterization of the targeted autoantigen.

Thus, the present invention also provides a kit for culturing and treating T cells (e.g., harvested from peripheral blood) with α-MSH in the presence of a specific antigen and antigen presenting cells. After incubation, the α-MSH-treated T cells can be collected and injected back into the patient. The kit comprises α-MSH, a specific, target antigen, and an article of manufacture comprising instructions for how to use the α-MSH and target antigen to generate regulatory T cells. Optionally, the kit may also include T cell culture media conducive to generation of the CD4+/CD25+, TGF-β-producing T cells of the invention. TGF-β2 is also included in the kit. The target antigen can be an autoantigen, so as to generate a regulatory T cell that specifically recognizes the autoantigen and reestablishes tolerance to that antigen. Alternatively, the target can be a 'surrogate' antigen, which would still generate regulatory T cells of the invention that are effective for down-regulating an autoimmune response or a host-versus-graft response, in that the regulatory T cells would still produce TGF-β and other cytokines necessary to down-regulate a T cell-mediated inflammatory response. Preferably, at least two samples of the target antigen should be included in the kit, one sample for adding to the T cell cultures in which regulatory T cells are to be generated, and one sample for injection into the diseased tissue site of the patient or the transplantation site of the graft recipient.

The experimental results presented herein, demonstrate that a population of T cells expressing on their surface the cell surface proteins CD4 and CD25, expand when activated in the presence of α-MSH. Such CD4⁺ / CD25⁺ T cells are known to be regulatory in activity, and thus seem to be the regulatory T cells induced by α-MSH in the method of the present invention. This observation suggests that a loss in peripheral blood CD4/CD25-positive cells, in response to presented autoantigen, could indicate a patient's susceptibility to a specific autoimmune disease. Therefore, the level of CD4/CD25-positive T cells in peripheral blood could serve as a prognostic indicator for an individual's susceptibility to autoimmune disease or relative risk of rejecting a transplant, and could also be used to asses the effectiveness of α-MSH treatment in that individual.

### BRIEF DESCRIPTION OF THE DRAWINGS

Figure 1 is a bar graph of primed T cell proliferation, as measured by H³-thymidine uptake (CPM), in response to T cell receptor stimulation (anti-CD3) in the presence of various concentrations of α-MSH (pg/ml);
Figure 2 is a bar graph showing the extent of IFN-γ production by primed T cells TCR-stimulated in the presence of α-MSH;
Figure 3 presents the results of flow cytometric analysis of intracellular IFN-γ and IL-4 production by primed T cells TCR-stimulated in the presence of α-MSH;
Figure 4 is a bar graph of TGF-β production (pg/ml) by primed T cells TCR-stimulated in the presence of various concentrations of α-MSH (pg/ml);
Figure 5 is a bar graph showing production of IFN-γ by primed T cells in response to TCR stimulation alone or in the presence of α-MSH;
Figure 6 is a bar graph similar to Figure 5, but shows the long-term effect of α-MSH treatment on the TCR-stimulated primed T cells (IFN-γ production in response to TCR re-stimulation on day 5 after α-MSH treatment);
Figures 7A and 7B show the results of SDS-PAGE analysis of primed T cell lysates after incubation under various conditions: with or without α-MSH and with or without TCR stimulation, followed by immunoblotting with anti-phosphotyrosine antibody;
Figure 8 depicts a bar chart showing DTH response in mice as measured by ear swelling, i.e., change in ear thickness (µm), as a function of the type of T cells administered, including whether or not the mice were injected with activated OVA-primed T cells treated with aqueous humor ("Regulatory T cells; AqH/OVA");
Figure 9 is a graph plotting percent proliferation of T cells in response to varying concentrations of TGF-β1 or TGF-β2, with or without α-MSH;
Figure 10 is a graph potting percent proliferation as a function of the time at which either TGF-β1 or TGF-β2 was added (Hours TGF-β Added), after TCR-stimulation in the presence of α-MSH;
Figure 11 is a bar chart showing total TGF-β production in cells treated with α-MSH and either TGF-β1 or TGF-β2, as a function of the time at which the TGF-β was added;
Figure 12 shows the percent suppression in IFN-γ in activated effector T cells. TGF-β and α-MSH induce regulatory T cell activity;
Figure 13 depicts a bar chart showing DTH response in mice as measured by ear swelling, i.e., change in ear thickness (µm), as a function of the type(s) of T cells administered, including whether or not the mice were injected with activated, OVA-primed T cells treated with α-MSH and TGF-β2 ("Regulatory T cells; α-MSH / TGF - β2 OVA");
Figure 14 shows the mean uveitis score in α-MSH-treated and untreated mice afflicted with experimental autoimmune uveitis (EAU).

### DETAILED DESCRIPTION OF THE INVENTION

Regulation of T cell activity is needed for maintaining tolerance to autoantigens. One of the regulatory mechanisms is mediated by factors produced by cells within a tissue microenvironment. One of these regulating factors is the neuropeptide, α-melanocyte stimulating hormone (α-MSH), which is shown by the work herein to suppress immunogenic inflammation. As demonstrated in Example I, Th1 cells are suppressed from mediating inflammation when they are activated in the presence of α-MSH. Although there is T cell proliferation, α-MSH suppresses IFN-γ production and possibly the secretion of IL-4 by T cell receptor (TCR)-stimulated, primed T cells. Such α-MSH treated T cells produce enhanced levels of TGF-β. These TGF-β-producing T cells are characteristic of Th3 cells, and suppress IFN-γ production by other, activated Th1 cells. Therefore, it is showon here that α-MSH suppression of Th1 cell activity results from α-MSH apparently deviating the Th1 response into a Th3-like response. α-MSH enhances tyrosine phosphorylation of CD3ζ chains but not of CD3ε chains. This phenomenon suggests that α-MSH could mediate immune response deviation through induction of differential, TCR-associated signals in T cells. The ability of α-MSH to mediate induction of TGF-β-producing, regulatory T cells implies that this neuropeptide has an important systemic and regional role in mediating and maintaining peripheral tolerance, especially in tissues such as the eye and the brain where α-MSH is constitutively present.

The ocular microenvironment is an extreme example of regional immunity. Within its microenvironment, expression of delayed type hypersensitivity (DTH) is suppressed. This immunosuppression is mediated in part by the constitutive expression of α-MSH in aqeuous humor. α-MSH has been found to suppress the production of IFN-γ by activated effector T cells (Th1).

The experiments of Example II were undertaken to determine whether aqueous humor-induced regulatory T cells could function *in vivo*. These regulatory T cells were examined for their ability to suppress adoptive transfer of delayed-type hypersensitivity (DTH). In addition, two aqueous humor factors, α-MSH and TGF-β2, were examined for their respective ability to induce regulatory T cells.

Primed T cells were treated with aqueous humor, α-MSH, TGF-β1, or TGF-β2 in Example II. These treated T cells were assayed for regulatory activity by injecting them intravenously (i.v.) along with inflammatory Th1 cells into syngeneic mice. Antigen-pulsed, antigen presenting cells (APC) were injected into the pinna of the mouse ear and swelling was measured 24 hours later. Primed T cells were also activated *in vitro* in the presence of α-MSH, TGF-β1 or TGF-β2, and were assayed for proliferation and TGF-β production along with their ability to suppress DTH.

The Example II results show that aqueous humor-treated T cells suppressed DTH mediated by Th1 cells. Maximum regulatory T cell activity was induced when primed T cells were activated *in vitro* in the presence of α-MSH, followed approximately 4 hours later with addition of active TGF-β2. Such T cells proliferated and produced TGF-β, suggesting that α-MSH and TGF-β2 induced activation of Th3 cells. No regulatory T cell activity could be induced in the presence of TGF-β1 (alone or in the presence of α-MSH). Therefore, not only do α-MSH and TGF-β2 have direct immunosuppressive effects. Additionally, through the constitutive production of α-MSH and TGF-β2, the ocular microenvironment can mediate induction of regulatory T, possibly Th3, cells that can contribute further to the immunosuppressive microenvironment and immune privilege of the eye, through their production of TGF-β and by their ability to suppress activation of Th1 cells. Such a mechanism of immunosuppression may mediate the peripheral tolerance to ocular antigens that is needed to prevent induction of ocular autoimmune diseases.

In light of the finding, in Example I, that α-MSH can mediate induction of TGF-β-producing, regulatory T cells, the experiments in Example III were conducted to examine α-MSH's ability to suppress T cell-mediated inflammation (e.g., as in autoimmune disease) and to regulate lymphokine production by effector T cells. When α-MSH was injected intravenously (i.v.) into mice at the time of peak retinal inflammation, the severity of experimental autoimmune uveitis (EAU) was significantly suppressed. Effector T cells that were activated *in vitro* in the presence of α-MSH, proliferated and produced IL-4 as well as enhanced levels of TGF-β. However, their IFN-γ and IL-10 production was suppressed. The α-MSH-treated T cells functioned as regulatory T cells by suppressing *in vitro* IFN-γ production by other inflammatory T cells. This regulatory activity was the function of α-MSH-treated, CD4⁺ CD25⁺ T cells. Therefore, α-MSH mediates immunosuppression by inducing a differential expression of lymphokine production and by inducing activation of regulatory functions in T cells. This implies that α-MSH may take part in regional mechanisms of immunosuppression and possibly peripheral tolerance. Thus, α-MSH can be used to suppress autoimmune disease and possibly to re-establish tolerance to autoantigens.

The invention is described further by way of the following, non-limiting examples.

### MATERIAL AND METHODS

**Antibodies and Cytokines.** For TCR-stimulation, anti-CD3ε antibody 145-2C11 from_Pharmingen (San Diego, CA) was used at a concentration that stimulated maximum proliferation and IFN-γ production by the primed Th1 cells (see below). In the sandwich ELISAs, capture antibody BVD4-1D11 and biotinylated-detection antibody BVD6-24G2 were used for the IL-4 assay; and the IFN-γ assay used capture antibody R4-6A2 and biotinylated-detection antibody XMG1.1, all from Pharmingen. Recombinant mouse lymphokines IL-4 (R&D Systems, Minneapolis, MN) and IFN-γ (Genzyme, Cambridge, MA) were used for standards in the sandwich ELISAs. For flow cytometry, Pharmingen's PE-conjugated anti-CD4 antibody and FITC-conjugated antibodies BVD4-1D11 (anti-IL-4) and XMG1.1 (anti-IFN-γ) were used. Synthesized α-MSH was from Peninsula Laboratories (Belmont, CA), and purified human TGF-β2 was from R&D systems. In addition, Santa Cruz Biotechnologies (Santa Cruz, CA) anti-CD3ζ antibody and anti-CD3ε antibody plus rabbit anti-hamster IgG antibody (Sigma) were used in immunoprecipitation and anti-phosphotyrosine antibody PY20 (ICN, Costa Mesa, CA) for immunoblotting.

**α-MSH treatment of *in vivo* primed T cells.** *In vivo* primed T cells were obtained from BALB/c mice (institute breeding program) immunized via a cutaneous foot injection with 0.5 mg desiccated *Mycobacterium tuberculosis* (Difco, Detroit, MI). From the draining popliteal lymph node, the T cells were enriched, 99% CD3⁺ by flow cytometry analysis, using a mouse T cell enrichment column (R&D Systems). Into the wells of a 96 well, round bottom plate (Corning, Corning, NY) were added 100 µl of T cells (4 x 10⁶ cells/ml), 50 µl of α-MSH (30 pg/ml) and 50 µl of 2C11 antibody (1 µg/ml) in serum-free culture media. The cultures were incubated for 48 hours and lymphokines in the culture supernatant were assayed using sandwich enzyme linked immunosorbent assays (ELISA) specific for IFN-γ and IL-4 and using the standard CCL-64 bioassay for TGF-β. The serum-free culture media (23) was RPMI 1640, 1/75 dilution of sterile 7.5 % BSA solution (Sigma Chemical, St. Louis, MO), 1/500 dilution of ITS+ solution (Collaborative Biomedical Products, Bedford, MA). For assaying proliferation, the T cell cultures were initially incubated for 24 hours; then 20 µl of 50 µCi/ml of 3H-thymidine (NEM, Boston, MA) were added to the wells and the cultures were incubated for an additional 24 hours. The cells were collected onto filter paper using a Tomtec Plate Harvester 96 and radiolabel was measured using a Wallac 1205 Betaplate Liquid Scintillation Counter.

**Sandwich Enzyme Linked Immunosorbent Assay.** The wells of a 96-well flexible microtiter plate (Falcon, Oxnard, CA) were coated with 50 µl of 1 µg/ml capturing monoclonal antibody (anti-IFN-γ or anti-IL-4) in 50 mM carbonate/bicarbonate buffer (pH 9.6) (Sigma Chemical). The plate was incubated overnight at 4°C and washed once with 10 mM phosphate buffer saline (PBS), 0.02% Tween-20 (wash buffer). The wells were blocked by washing the wells three times with 200 µl of Superblock (Pierce, Rockford, IL). To the wells, 100 µl of condition media was added and the plate was incubated 2 hours at room temperature. The wells were washed three times with wash buffer and into each well 100 µl of 1.0 µg/ml of biotinylated-detecting monoclonal anti-IFN-γ or anti-IL-4 antibody was added. The plate was incubated for 1 hour and washed three times with wash buffer followed by the addition to each well 100 µl of 1 mg/ml strepavidin-β-galacotsidase (Gibco/BRL, Gaithersburg, MD). The plate was incubated for 30 minutes, washed 5 times with wash buffer, and 100 µl of 1 mg/ml of chlorophenylred-b-d-galactoside (CPRG: Calbiochem, San Diego, CA) in 0.05 M PBS with 1.5 mM MgCl2 was added. Color was allowed to develop for 1 - 2 hours and the optical density of the converted CPRG was read on a Tecan SLR ELISA plate reader at a wavelength of 570nm. Using a standard curve created from the OD of wells on the same plate containing known concentrations of the lymphokine, the concentration of lymphokine in the assayed culture supernatants was calculated.

**TGF-β bioassay.** To measure total TGF-β, 100 µl of conditioned media was pretreated according to standard procedures (24) with 10 µl 1N HCL to lower the media pH to 2 and incubated for 1 hour at 4°C. The acid was neutralized with 20 µl of a 1:1 mixture of 1N NaOH: 1M HEPES returning the culture supernatant to a pH 7.3. The transiently acidified conditioned media was then used in the Mv1Lu assay; diluted 1:4 in EMEM + 0.5% FBS. To the wells of a Falcon 96-well flat-bottom plate, 100 µl of diluted transiently acidified samples were added with 100 µl of 1 x 10⁵ Mv1Lu cells (CCL-64; ATCC, Rockville, MD). The plate was incubated for 20 hours at 37°C, 5% CO₂ followed by the addition of 20 µl of 50 µCi/ml 3H-thymidine and the plate was incubated for an additional 4 hours. Supernatant was discarded and 50 µl of 10x Trypsin-EDTA (BioWhittaker, Walkersville, MD) solution was added to each well and the plate was incubated for 15 minute at 37°C. The cells were collected onto filter paper using a Tomtec Plate Harvester 96 and counts per minute (CPM) of incorporation 3H-thymidine was measured using a Wallac 1205 Betaplate Liquid Scintillation Counter. Cultures of known amounts of purified activated TGF-β1, 20 ng/ml to 0.2 pg/ml (R&D Systems), were prepared in the same plate for calculating a standard curve to quantify the concentration of total TGF-β in the samples.

**Flow Cytometry.** For immunostaining and flow cytometry, T cells (2 x 10⁶ cells) were obtained from 24 hour cultures of enriched primed T cells TCR-stimulated in the presence of α-MSH as described above. The cells were centrifuged and washed once in 400 µl of brefeldin/PBS buffer (10 mM PBS, 10 µg/ml brefeldin A). The cells were resuspended in 100 µl of PBS/brefeldin and 100 µl of 4% paraformaldehyde/PBS fixing buffer. The cells were incubate at room temperature for 20 minutes with gentle agitation and washed once with 200 µl of brefeldin/PBS, centrifuged, and resuspended in 50 µl of PBS/saponin (10 mM PBS, 1% BSA, 0.1% Na Azide, 0.5% Saponin) and incubated 10 minutes at room temperature. To the cell suspensions 2 µg of FITC-conjugated anti-cytokine antibody (anti-IL-4 or -IFN-γ) or FITC-isotype control was added. The cells were incubated for 30 minutes at room temperature, washed twice with PBS/saponin, and washed once with PBS/BSA buffer (10 mM PBS, 3% BSA). The cells were resuspended in 50 ml of PBS/BSA buffer containing 2 µg of PE-conjugated anti-CD4 antibody and incubated for 30 minutes room temperature. The cells were centrifuged, resuspended in 1 ml of PBS/BSA buffer, and strained through nylon mesh into a snap cap tube with an additional 1 ml of PBS/BSA buffer washed through the mesh. The stained cells were analyzed by a Coulter Epics flow cytometer calibrated for two color fluorescence, and presented were the fluorescence of blast (proliferating) cells in two dimensions.

**Immunoprecipitation and Immunoblotting.** Enriched Th1 cells (2 x 10⁶ cells) in a 24-well Corning plate were TCR-stimulated with 2C11 in the presence of α-MSH (30 pg/ml) under serum-free conditions for 15 minutes. The T cells were collected placed in a microcentrifuge tube and washed once with 10 mM Tris buffered saline (TBS) and lysed for 30 minutes in 100 µl of ice cold lysate buffer (10 mM TBS, 1% NP-40, 0.5% sodium deoxycholate, 0.1% SDS, 100 µg/ml phenylmethylsulfonyl fluoride (PMSF), 60 µg/ml Aprotinin, and 1 mM sodium orthovanadate). The cells were passed through a 21 gauge needles three times, and an additional 2 µl of 10 mg/ml PMSF was added. The tubes were incubated for an additional 30 minutes on ice and were centrifuged for 20 minutes, 4°C, 13,000 x g. The supernatant was collected and used as total cellular lysate. To the lysates were added 20 µg/ml of anti-CD3ζ antibody, or anti-CD3ε antibody plus rabbit anti-hamster IgG antibody (which was needed for the CD3ε immunoprecipitation since much of the CD3ε was bound by 2C11 a hamster anti-mouse CD3ε and hamster antibody weakly binds protein-G), and incubated overnight at 4°C. Protein-G sepharose beads (Pharmacia, Piscataway, NJ) were added to the antibody-containing lysates and incubated for 1 hour at room temperature with end-over-end agitation. The beads were centrifuged and washed 4 times with 10 mM TBS containing 0.5% sodium deoxycholate. After the final wash the beads were resuspended in 20 µl of distilled water and 20 µl non-reducing SDS Tris-glycine sample buffer (Novex, San Diego, CA), boiled for 5 minutes and applied into two wells (20 µl) of a 8-16% Tris-glycine polyacrylamide gel (Novex). Following electrophoresis in a Novex Xcell II minicell and blot module, the proteins were transferred from the gel onto a nitrocellulose membrane (Novex) by electroblotting. The membrane was blocked with 1% BSA in 0.01 M TBS for 1 hour room temperature. The blocked membrane was cut into two pieces. One piece was placed in a sealable bag containing 5 ml of alkaline-phosphatase conjugated anti-phosphotyrosine antibody PY20 diluted 1/2000 in 1% BSA-TBS buffer. The other membrane piece was placed into a sealable bag containing 5 ml of anit-CD3ζ or anti-CD3ε antibody diluted 1/100 in 1% BSA-TBS buffer respective to the immunoprecipitation. Filters were incubated overnight at room temperature. The PY20 blotted membrane was washed 3 times with wash buffer containing 1% BSA-TBS and 0.05% Tween-20 and incubated with alkaline phosphatase substrate NBT-BCIP (Sigma Chemical) until bands appeared. The membrane was washed with distilled water. The anti-CD3 protein blotted membranes were washed 3 times with 1% BSA-TBS and placed into a new sealable bag containing 5 ml of alkaline phosphatase conjugated anti-mouse IgG (Sigma Chemical) diluted 1/ 10,000 in 1% BSA-TBS buffer and incubated for 1 hour room temperature. The membranes were washed 3 times with wash buffer and incubated with substrate until bands appeared and washed. Membranes were then digitally photographed and analyzed using NIH Image software to integrating the band intensity to band area minus background.

**Assay For *In Vitro* Regulatory T Cell Activity.** The α-MSH-treated TCR-stimulated T cells, as described above, were cultured for 48 hours. The plate was spun down at 250 x g for 10 minutes and supernatant discarded. Freshly isolated, enriched *in vivo* primed Th1 cells (4 x 10⁶ cells/ml) mixed with 2C11 (1 µg/ml) were added (200 µl) to the wells of the α-MSH pre-treated, TCR-stimulated T cells and incubated for 48 hours. The culture supernatant was assayed for IFN-γ by sandwich ELISA. For long term cultures, primed T cells were TCR-stimulated in the presence of α-MSH for 48 hours. The plate was centrifuged and supernatant was exchanged for 200 µl of 2C11 (1 µg/ml) in fresh media, no α-MSH. The cultures were incubated for 72 hours and the conditioned media was again exchanged for fresh media and 2C11 antibody. The cultures were incubated for an additional 48 hours, centrifuged, supernatant discarded, and added were fresh *in vivo* primed Th1 cells and 2C11 antibody. These cultures were incubated for 48 hours and the culture supernatant was assayed for IFN-γ by sandwich ELISA.

### EXAMPLE I RESULTS

### α-MSH Has No Effect On TCR-Stimulated T Cell Proliferation.

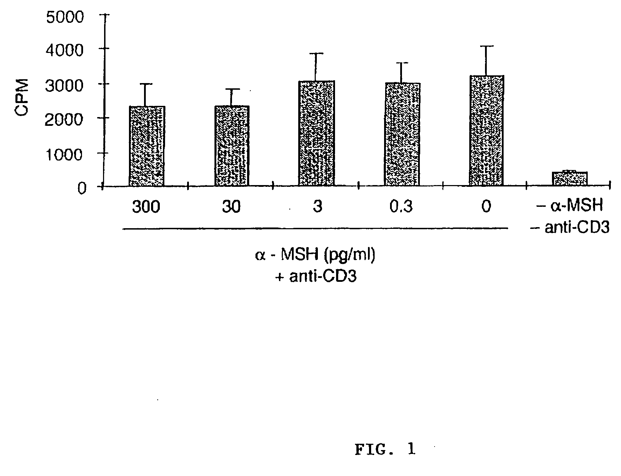

Since α-MSH was previously found to suppress IFN-γ production by antigen-stimulated Th1 cells (9), we investigated whether α-MSH suppresses all TCR-associated activities or only IFN-γ production. Additionally, α-MSH has been shown to have the potential to affect both antigen presenting cells (APC) and T cells. This observation made it uncertain as to whether the T cells could be a direct target of α-MSH immunosuppressive activity. To eliminate the influence of α-MSH on APC activation of T cells, APC were removed by enriching for CD3⁺ T cells from lymph nodes primed to *M. tuberculosis.* Also, the T cells were stimulated with anti-CD3ε antibody, 2C11, at a concentration that stimulated the T cells to maximally proliferate and produce IFN-γ, a maximized *in vitro* Th1 cell response. To examine the possibility that our previously observed α-MSH suppression of INF-γ production was due to α-MSH suppression of Th1 cell activation, the enriched primed T cells were TCR-stimulated in the presence of α-MSH and proliferation was assayed. We found that α-MSH had no effect on TCR-stimulated Th1 cell proliferation (Figure 1). Therefore, α-MSH has no effect on the TCR-associated signals mediating proliferation.

Figure 1, depicts the proliferation of primed T cell (H³-thymidine uptake (CPM)), in response to T cell receptor stimulation (anti-CD3) in the presence of various concentrations of α-MSH (pg/ml). T cells from a draining lymph node of a BALB/c mouse immunized 7 days previously with *M. tuberculosis* were enriched and incubated (4 x 10⁵ cells) in serum-free media with anti-CD3ε (2C11) in the presence of declining concentrations of α-MSH for 24 hours. To the cultures was added 0.50 µCi of ³H-thymidine and they were incubated for an additional 24 hours. The cells were collected and counted by scintillation for incorporated radiolabel. The Fig. 1 results are presented as CPM ± SEM of eight independent experiments.

### α-MSH Suppresses IFN-γ production and IL-4 secretion by TCR-stimulated Primed T cells.

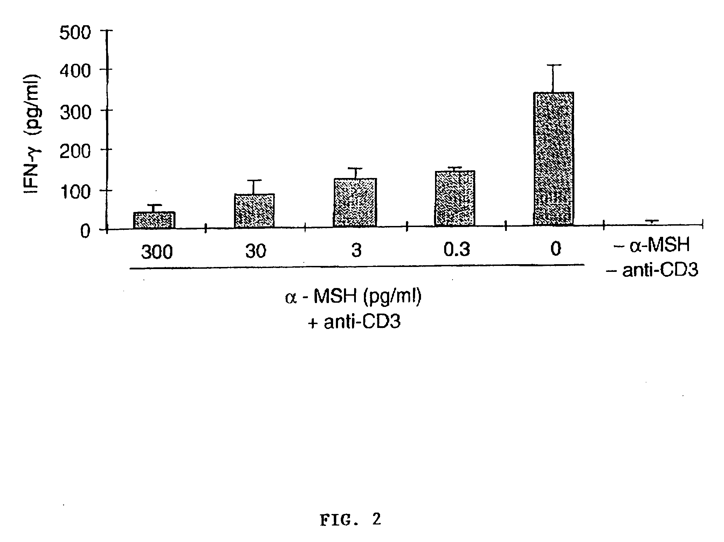

Since TCR-stimulated proliferation was not affected by α-MSH, it is possible that α-MSH affects selective TCR-associated activities. We assayed the culture supernatant of the enriched primed Th1 cells TCR-stimulated in the presence of α-MSH for INF-γ and IL-4. IFN-γ production was suppressed when the primed T cells were TCR-stimulated in the presence of physiological concentrations of α-MSH, as shown in Figure 2.

Figure 2 is a bar graph showing the extent of IFN-γ production by primed T cells TCR-stimulated in the presence of α-MSH. Primed T cells were obtained, enriched and cultured as in Figure 1 and incubated for 48 hours. The culture supernatants were assayed for IFN-γ by sandwich ELISA. The results are presented as pg/ml ± SEM of eight independent experiments. In all assayed concentrations of α-MSH, levels of IFN-γ in the culture supernatant were significantly (p = 0.05) suppressed in comparison to the levels of IFN-γ in the cultures of enriched primed T cells TCR-stimulated in the absence of α-MSH.

Also assayed was IL-4, since its production is considered an indication of Th2 cell activity countering Th1 IFN-γ production. We could not find any IL-4 in the supernatants of any TCR-stimulated T cell cultures (data not shown).

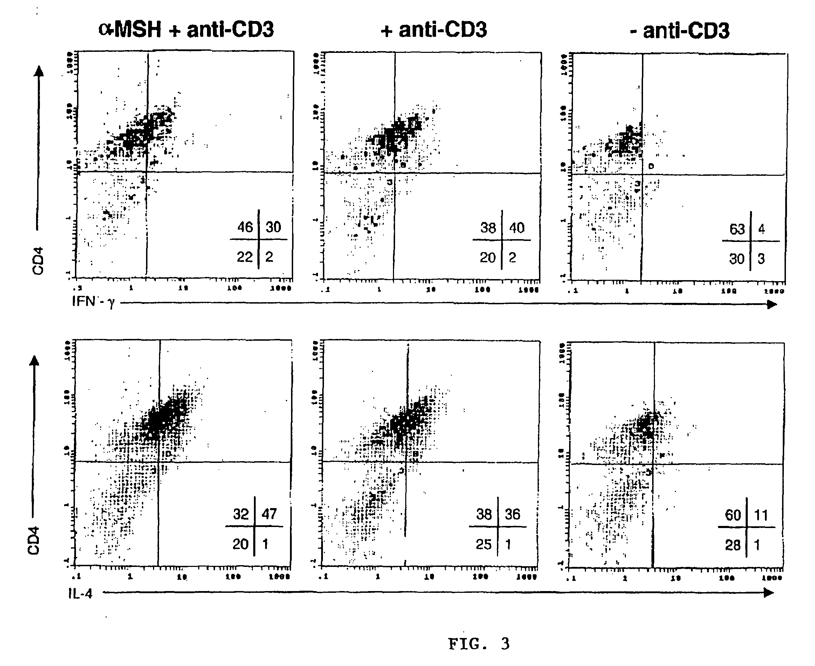

To further examine α-MSH suppression of IFN-γ production by T cells, the frequency of IFN-γ producing T cells activated in the presence of α-MSH was assayed by flow cytometry. The α-MSH treated TCR-stimulated T cells were stained for surface-expressed CD4 and for intracellular IFN-γ protein. The frequency of IFN-γ positive cells substantially shifted toward lower levels of intracellular IFN-γ staining in CD4⁺ T cells activated in the presence of α-MSH, as shown in Figure 3. Figure 3 presents the results of flow cytometric analysis of intracellular IFN-γ and IL-4 production by primed T cells TCR-stimulated in the presence of α-MSH. Primed T cells were obtained and enriched as in Figure 1. The enriched T cells (2 x 10⁶ cells) were incubated with anti-CD3 in the presence of α-MSH (30 pg/ml) for 24 hours. Unstimulated, enriched T cells were cultured in media alone (- anti-CD3). The T cells were fixed with paraformaldehyde, permeabilized with saponin and stained for intracellular IFN-γ or IL-4 with PE-conjugated antibodies in saponin buffer. The surface of the cells were stained with FITC-conjugated anti-CD4. The stained cells were analyzed by two-color flow cytometry gating on the blastogenic, proliferating T cells. Quadrant lines were placed to separate CD4⁺ and CD4⁻ cells vertically and PE-conjugated isotype control on the horizontal. The cell density was equilibrated among the histograms. The histograms are all from one experiment representing similar results of four independent experiments. Percent of analyzed cell population is given for each quadrant in the lower right-hand of each histogram. The presence of IFN-γ stained T cells was limited to only the CD4⁺ T cells. Therefore, the decrease in IFN-γ detected in the culture supernatants was due to a decrease in the frequency of IFN-γ-synthesizing T cells stimulated in the presence of α-MSH.

Even though no IL-4 was detected in the culture supernatant by ELISA, there was a shift to higher levels of IL-4 staining in the CD4⁺ T cells stimulated in the presence of α-MSH (Figure 3). Thus, the primed T cells activated in the presence of α-MSH expressed intracellular IL-4, but no IL-4 was detected in the culture supernatants. This observation suggests that α-MSH may promote induction of IL-4 protein synthesis, but that IL-4 secretion may be suppressed within at least the time span of these experiments (48 hours). Therefore, primed Th1 cells TCR-stimulated in the presence of α-MSH are suppressed in their IFN-γ production and are not fully deviated to a Th2 cell response.

### α-MSH Enhances Production of TGF-β by Primed T cells.

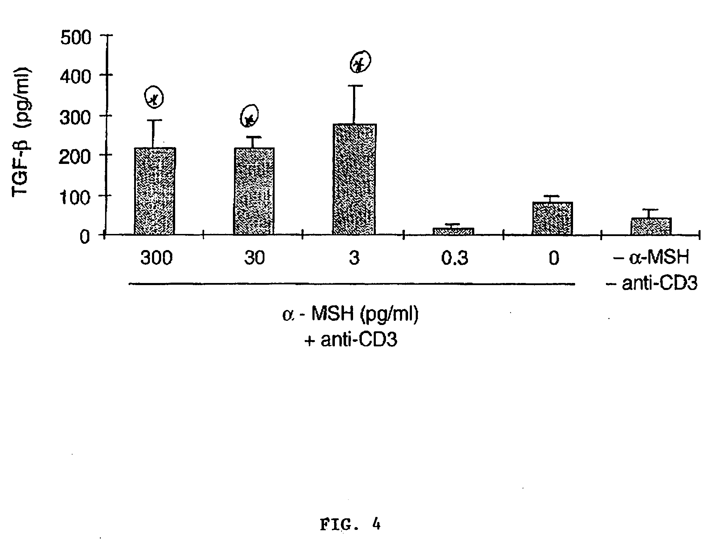

The oral tolerance models have suggested that TGF-β producing T cells have an important role in regulating and suppressing autoimmunity (25-29). It has been suggested that they be considered a third type of T cells, to contrast their suppressive activity with the immune functions of Th1 and Th2 cells. Since the primed T cells TCR-stimulated in the presence of α-MSH did not produce or release the prototypical lymphokines for Th1 (IFN-γ) and Th2 (IL-4) cells, experiments were conducted to determine whether the α-MSH treated primed T cells could produce the Th3-associated lymphokine, TGF-β (30). The enriched primed T cells were TCR-stimulated in the presence of α-MSH as before, and the culture supernatants were assayed for total TGF-β using the TGF-β bioassay (Figure 4).

Figure 4 is a bar graph of TGF-β production by primed T cells TCR-stimulated in the presence of α-MSH. Primed T cells were obtained, enriched and cultured as in Figure 1 under serum free conditions. After 48 hours of incubation the culture supernatant was collected and all TGF-β in the supernatant was activated by transiently acidified with 1N HCl neutralized by a 1:1 mixture of 1M HEPES: 1M NaOH. The transiently acidified supernatant was assayed for TGF-β using the standard mink lung endothelial cell bioassay. The results represented as TGF-β pg/ml ± SEM of eight independent experiments. Significance (p = 0.05) was determined by Student's t-test between TGF-β concentrations in cultures of activated α-MSH treated T cells and T cells activated in the absence of α-MSH.

Thus, TGF-β production was significantly enhanced in cultures of α-MSH-treated, TCR-stimulated primed T cells. Therefore, when *in vivo*, primed Th1 cells that are TCR-stimulated in the presence of α-MSH produce TGF-β, possibly IL-4, but not the expected IFN-γ. Such T cells are generally considered to be Th3 cells.

### α-MSH Mediates Induction Of Regulatory T Cells.

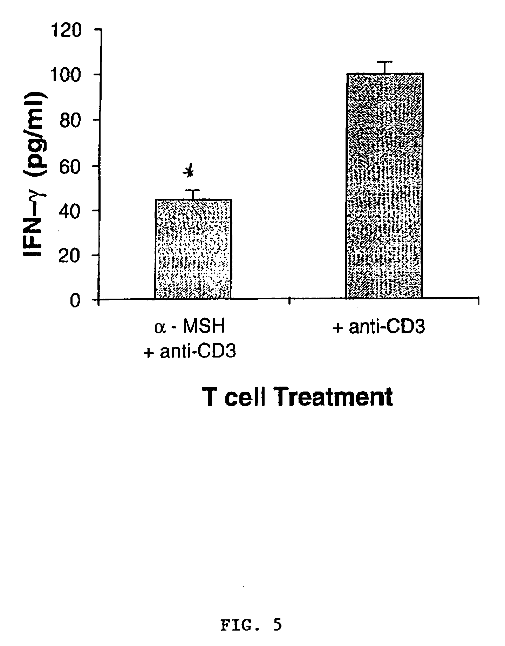

If α-MSH is mediating induction of Th3 cell function, then these α-MSH treated T cells should be regulatory in activity. The T cells were TCR-stimulated in the presence of α-MSH as before, incubated, and then mixed with fresh TCR-stimulated primed Th1 cells. The amount of IFN-γ produced in cultures of activated primed Th1 cells was suppressed by 60% percent when the α-MSH treated T cells were mixed into the cultures, as shown in Figure 5.

Figure 5 shows the effect on primed T cell production of IFN-γ, of TCR stimulation alone or in the presence of α-MSH. α-MSH induces regulatory activity in TCR-stimulated primed T cells. Primed T cells (4 x 10⁵ cells) were enriched and activated with anti-CD3 in the presence (α-MSH + anti-CD3) or absence (+ anti-CD3) of α-MSH (30 pg/ml). After 48 hours of incubation the cells were mixed with freshly enriched primed Th1 cells (4 x 10⁵ cells) and anti-CD3 antibody and cultured for 48 hours. The culture supernatant was assayed for IFN-γ by sandwich ELISA. Results are presented as IFN-γ pg/ml ± SEM of eight independent experiments. Concentration of IFN-γ in cultures containing T cells activated in the presence of α-MSH was significantly suppressed (p = 0.05) in comparison to cultures where none of the T cells had seen α-MSH.

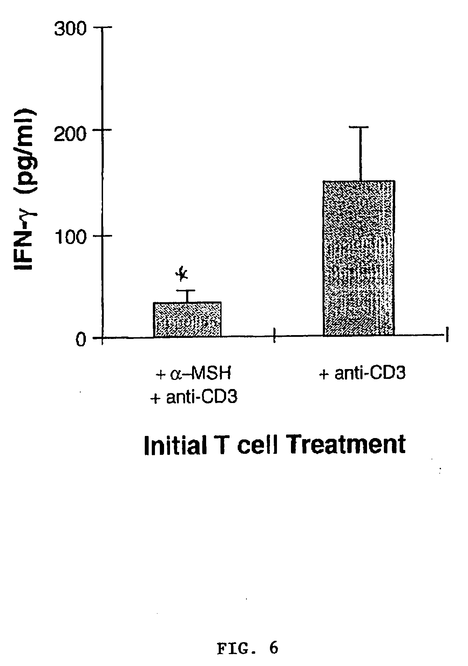

In addition, T cells initial TCR-stimulated in the presence of α-MSH and subsequently restimulated twice (Day 2 and Day 5) with anti-TCR antibody in the absence of α-MSH, still suppressed IFN-γ production by freshly activated primed Th1 cells (Figure 6).

Figure 6 shows that α-MSH induces long term regulatory activity in TCR-stimulated primed T cells. Primed T cells (4 x 10⁵ cells) were enriched and activated with anti-CD3 antibody in the presence of 30 pg/ml α-MSH (α-MSH + anti-CD3) or absence of α-MSH (+ anti-CD3). After 48 hours of incubation the cells were centrifuged and resuspended in fresh media with anti-CD3 antibodies and incubated for 72 hours (day 5). The cells were again washed and re-stimulated with anti-CD3 and incubated for an additional 48 hours (day 7). After the final incubation the treated T cells (4 x 10⁵ cells) were mixed with freshly enriched primed Th1 cells (4 x 10⁵ cells) and anti-CD3 antibody and cultured for 48 hours. The culture supernatant was assayed for IFN-γ by sandwich ELISA. Results are presented as IFN-γ pg/ml ± SEM of eight independent experiments. Concentration of IFN-γ in cultures containing T cells initially activated in the presence of α-MSH was significantly (p = 0.05) suppressed in comparison to cultures where none of the T cells had seen α-MSH. This observation suggests that α-MSH induces a permanent and stable regulatory function in the T cells. Therefore, α-MSH mediates induction of functionally active, regulatory Th3 cells.

### α-MSH Enhances CD3ζ Chain Phosphorylation In Activated Primed T-Cells.

Since the induction of lymphokine production is linked to TCR-stimulation, there is a possibility that intracellular signals, emanating from the α-MSH-engaged melanocortin receptor, influence the tyrosine phosphorylation of CD3 molecules of the TCR. Such an influence could induce differential TCR-dependent responses (31, 32). To demonstrate that α-MSH affects T cell response to TCR-stimulation, lysates of primed T cells TCR-stimulated in the presence of α-MSH were immunoprecipitated with either antibodies against CD3ζ or CD3ε chains, electrophoresed, and immunoblotted for phosphotyrosine.

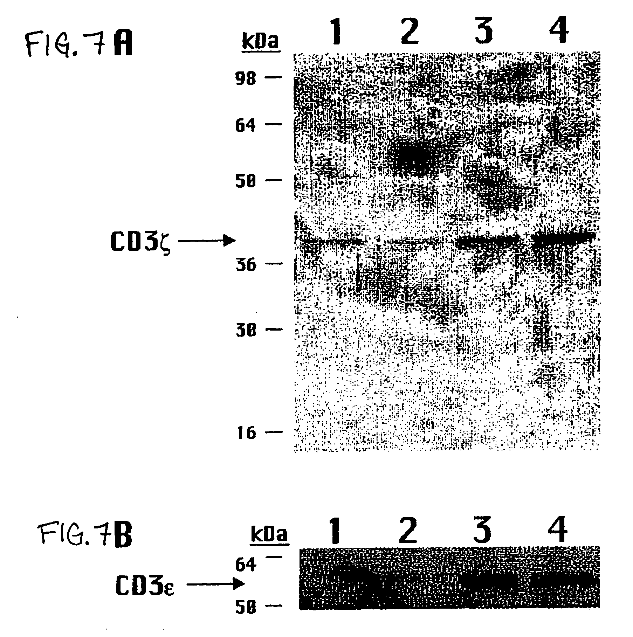

The results are shown in Figures 7A-B, and demonstrate the tyrosine phosphorylation of CD3ε and CD3ζ in primed T cells TCR-stimulated in the presence of α-MSH. The enriched primed T cells (2 x 10⁶ cells) were incubated with anti-CD3 in the presence of α-MSH (30 pg/ml) for 15 minutes. Unstimulated enriched T cells were cultured in media alone. The cells were washed and lysed. The lysates were immunoprecipitated with either anti-CD3ζ antibodies (A) or anti-CD3ε antibodies (B). The immunoprecipitates were analyzed by non-reducing SDS-PAGE (on a 8 - 16% gradient gel), followed by transfer to a nitrocellulose filter and immunoblotting with anti-phosphotyrosine antibody: (1) Unstimulated primed T cells treated with α-MSH; (2) Unstimulated primed T cells incubated in media alone; (3) Enriched primed T cells TCR-stimulated in the absence of α-MSH; (4) Enriched primed T cells TCR-stimulated in the presence of α-MSH. The CD3ζ dimers were detected at 42 kDa (CD3ζ-ζ) and the CD3ε heterodimers were detected at 55 kDa (CD3ε heterodimers) by a simultaneous immunoblot of unstimulated T cell lysates run on the same gel using the anti-CD3ζ or anti-CD3ε antibodies in the immunoprecipitation and immunoblot procedures. The relative intensities of the bands minus background, in lane order, for CD3ζ are 4, 1, 10, 67; and for CD3ε are 1, 1, 10, 10. The immunoblots are representative of three independent experiments.

The primed T cells, TCR-stimulated in the presence of α-MSH (Th3), had a 6.7-fold increase in CD3ζ tyrosine phosphorylation than the primed T cells stimulated in the absence of α-MSH (Th1 cells, Figure 7A). In addition, α-MSH stimulated tyrosine phosphorylation of CD3ζ dimers by 4 fold in unstimulated primed T cells (Figure 7A). The presence of α-MSH had no effect on the level of tyrosine phosphorylation of CD3ε chains of TCR-stimulated and unstimulated primed T cells (Figure 7B). Therefore, it is possible that the induction of Th3 cells by α-MSH is due to α-MSH mediating a TCR-associated signal that is independent of TCR engagement. The results also further indicate that *in vivo* primed Th1 cells are receptive to α-MSH, resulting in their deviation into Th3 cells.

### DISCUSSION OF EXAMPLE I

While the neuropeptide α-MSH suppresses Th1 cell responses, it induces regulatory activity in the same activated primed T cell population. Primed Th1 cells activated in the presence of α-MSH are suppressed in their expected production of IFN-γ and now produce TGF-β and possibly IL-4 indicating that the α-MSH has mediated a deviation of the activated Th1 cells into Th3 cells. Such T cells continue to proliferate and now function as immunoregulating T cells. It appears that α-MSH mediates some of its regulatory activity in T cells through differential tyrosine phosphorylation signals emanating from engaged T cell receptor proteins. The results here imply an important physiological role for α-MSH in regulating peripheral T cell activity. This role of α-MSH is important especially in tissues such as the brain and the eye where α-MSH is constitutively present.

The results show that α-MSH induces specific lymphokine production by the activated primed Th cells. Moreover, the results demonstrate that α-MSH can influence the functional developmental of primed Th cells. Such an observation falls more in line with the understanding that the functional differentiation of Th cells is mediated by their activation in the presence of specific cytokines, such as IL-12 for Th1 and IL-4 for Th2 development (29). Previously, α-MSH has been shown to act in a manner similar to IL-4, by suppressing induction of IFN-γ by TCR-stimulated Th1 cells (9). However, the present results indicate that the influence of α-MSH on Th cell functionality is not an IL-4-like mediated deviation to Th2, but an induction of a Th3 response. It has recently been found that induction of Th3 cells can be mediated by IL-4 along with the neutralization or absence of IL-12 (33). Therefore, the induction of a Th3 response could be mediated by α-MSH, acting on the Th cells as an IL-4-like agonist and as an IL-12 antagonist.

The potential for α-MSH to signal in lymphocytes in a manner similar to interleukins and other cytokines, has recently been found in B cells. There, α-MSH, through its melanocortin receptor (MC)-5, a G-protein associated receptor, activates the JAK1/STAT1 and STAT2 signal pathways (34). In this manner, α-MSH may act on T cells like other cytokines (i.e. IL-4) in regulating T cell development and differentiation into Th3 cells. It is not yet known which of the five melanocortin receptors are expressed on T cells and whether all the α-MSH receptors have a similar ability to activate JAK/STAT signal pathways (35). For intracellular signaling, the present results indicate that the α-MSH receptor must somehow interact with the TCR, leading to enhanced tyrosine phosphorylation of CD3ζ chains. The threshold of T cell activation is in relationship to the extent of phosphorylation of immune receptor, tyrosine-based, activation motifs (ITAM) on the TCR-CD3 proteins (31, 36). The extent by which the ITAMs are phosphorylated influences initiation of the differential signaling pathways emanating from TCR engagement (32, 36). The enhanced levels of CD3ζ chain tyrosine phosphorylation suggests that part of the effects of α-MSH on T cells, is through a signaling pathway of the TCR. It is possible that induction of regulatory Th cell activity by α-MSH is mediated through enhanced CD3ζ chain phosphorylation along with an α-MSH cytokine-like signal in the activated primed Th cells.

The result of activating *in vivo* primed Th1 cells in the presence of α-MSH is the induction of functional regulatory T cells. These T cells have been deviated by α-MSH away from their preset Th1 response (IFN-γ production). This deviation mediated by α-MSH may be an important function of α-MSH in tissues where there is a constitutive presence of α-MSH such as the eye and brain. The presence of bioactive α-MSH would promote the suppression of Th1 cells, including autoreactive T cells. Such α-MSH-mediated immunosuppression has been found in the immune privileged microenvironment of the eye (3, 23). Whether α-MSH is an important factor in modulating T cell activity in other immune privileged tissues is to be seen; however, there is evidence that α-MSH may be an important regulator of T cell functions in the brain (37).

The most dramatic characteristic of α-MSH treated, primed T cells is their production of TGF-β. The activation of such T cells would elevate TGF-β concentration within a localized tissue microenvironment. The elevated TGF-β concentration could influence the course of immune, inflammatory and wound-healing responses (38-44). The level of α-MSH activity in a tissue site could directly and indirectly, through TGF-β-producing T cells, regulate the induction, intensity, duration and resolution of an immune-mediated inflammatory response (38, 40). Therefore, by elevating the concentration of α-MSH in a tissue site enduring a DTH response, α-MSH would suppress the inflammation in part by repressing IFN-γ production by Th1 cells, and by inducing TGF-β production by Th3 cells.

α-MSH is does not merely suppress Th1 cell activity and inflammation. It also is potentially a mediator of T cell differentiation into Th3 cells(30). Also, like Th3 cells, the α-MSH-treated T cells suppress the inflammatory activity of other activated Th1 cells. Therefore, if such regulatory T cells are generated to a specific antigen there is the potential to induce antigen-specific tolerance. Since the α-MSH-rich fluid of the eye can induce induction of Th3 cells (45), it is possible that α-MSH mediates tolerance to ocular autoantigens through the induction of Th3 cells within the ocular microenvironment. It has already been demonstrated that a systemic elevation of α-MSH, through an i.v. injection, at the time of immunization, can induce antigen-specific tolerance (21). Therefore, since in the presence of α-MSH, activation of Th1 cells steers their development into CD4+/CD25+, Tgf-β-producing T cells (i.e., Th3 cells) that suppress the inflammatory activity of other activated Th1 cells, antigen-specific immunosuppression observed in the presence of α-MSH could very well be perceived as tolerance.

The immunosuppressive activity of α-MSH described here, suggests that if a tissue can be induced to secrete α-MSH, there would not only be prevention of an inflammatory response, but also the potential to induce immune tolerance to antigens within the tissue, through induction of antigen-specific Th3 cells. Therefore localized α-MSH treatment (12) into tissue and organ grafts may induce tolerance to the transplanted tissue antigens. It is also possible that if α-MSH is delivered into sites of autoimmune disease, there would be, along with suppression of inflammation, restoration of tolerance to the autoantigens through α-MSH-induced autoantigen-specific Th3 cells. This evolutionarily conserved neuropeptide, α-MSH, demonstrates a connection between the nervous and immune systems that can be exploited therapeutically to regulate antigen-specific immune responses.

### EXAMPLE II

### MATERIAL AND METHODS

### Mice.

B10.A and BALB/c mice (6-8 weeks old) were obtained from Jackson Laboratories (Bar Harbor, ME). Animals were treated in accordance with the ARVO Statement for the Use of Animals in Ophthalmic and Vision Research and the USA Federal Animal Welfare Act.

### Antigens.

Ovalbumin (OVA; Sigma Chemical, St. Louis, MO); desiccated *Mycobacterium tuberculosis* (MT-Ag; Difco, Detroit, MI) were used to immunize the mice.

### Aqueous Humor.

Aqueous humor (AqH) was obtained from New Zealand White rabbits (Pine Acres Rabbitry, West Brattleboro, VT) with no observed ocular and systemic disease. Aqueous humor was passively drained from the ocular anterior chamber by paracentesis through a 27 gage perfusion set (Fisher Scientific, Pittsburgh, PA) that ended in a siliconized microcentrifuge tube (Fisher Scientific). Collected aqueous humor was used immediately in the assays.

### T Cell Lines Specific for OVA.

B10.A mice were immunized with 1mg/ml OVA in complete Freund's adjuvant (Difco, Detroit, MI). After 7days, popliteal lymph nodes were collected and T cells were isolated using a mouse CD3 enrichment column (R&D systems, Minneapolis, MN). T cells were cultured with irradiated (2000R) spleen cells (5x10⁶ cells / well) from syngeneic B10.A mice in the presence of OVA (300µg/ml) for 7 days. The T cells were seeded at 2x10⁶ cells / well in a 24 well plate (Corning, Corning, NY) with completed Dulbecco's minimal essential medium (Biowhitter, Walkerville, Maryland) supplemented with 10% fetal bovine serum (Hyclone, Logan, Utah), 0.05mM 2-mercaptoethanol (Gibco/BRL, Grand Island, NY), 25mM HEPES (Biowhitter), 50µg/ml Gentamycin (Sigma Chemical), 5µg/ml L-Asparagine (Gibco/BRL), 5µg/ml L-Arginine (Gibco/BRL). The T cells were collected and restimulated with OVA and syngeneic irradiated spleen cells in the culture media containing 80 U/ml mouse recombinant IL-2 (R&D systems, Minneapolis, MN) and 4000 U/ml mouse recombinant IFN-γ (R&D systems). The T cells were restimulated with OVA once every 2 to 3 weeks in the presence of syngeneic spleen cells. Cultures of developing T cells were determined to be Th1 cells when the T cells produced only IFN-γ with no detectable IL-4 production when stimulated by OVA presenting APC in the absence of exogenous growth factors (IL-2, IFN-γ) and mediated inflammation in a standard adoptive transfer of DTH assay.

### Sandwich Enzyme Linked Immunosorbent Assay (ELISA).

The wells of a 96-well flexible microtiter plate (Falcon, Oxnard, CA) were coated with capturing monoclonal antibody (anti-IFN-γ; Pharmingen, San Diego, CA) to the cytokine being assayed. The plate was incubated overnight at 4°C and was washed with a solution of phosphate buffer saline, 0.02% Tween-20 and 1%BSA (wash buffer) and blocked with PBS plus 1% BSA (PBS-BSA). The plate was incubated for 1 hour room temperature and washed. Samples were added to the wells and the plate was incubated for 3 hours at room temperature. The plate was washed 3 times and into each well 100 µl of 1.0 µg/ml of biotinylated-detecting monoclonal anti-IFN-γ antibody (Pharmingen) was added. The plate was incubated for 1 hour and washed 3 times. Strepavidin-β-galactosidase (Gibco/BRL), 100 µl, was added to the wells and the plate was incubated for 30 min and washed 5 times. The substrate chlorophenyl-red-β-D-galactoside (CPRG: Calbiochem, San Diego, CA) was added to the wells and color was allowed to develop for 30 min. The optical density of the converted CPRG was read on a standard ELISA plate reader at a wavelength of 570nm. The INF-γ concentration of the standard samples were plotted against their OD to create a standard curve. Using this standard curve, the concentration of INF-γ in the assayed culture supernatant was determined from the OD of the test well and sample dilution factor.

### Adoptive transfer and Delayed-type hypersensitivity (DTH).

T cells from the draining lymph nodes of B10.A mice immunized 7 days previously with either OVA were isolated using a CD3 enrichment column (R&D Systems). The enriched primed T cells (4 x 10⁵ cells) were added to cultures containing aqueous humor (50% diluted in culture media) and antigen pulsed APC. The antigen pulsed APC were adherent spleen cells (1 x 10⁶ cells) from syngeneic naive mice pulsed overnight with antigen (OVA or MT-Ag) and washed with media before adding T cells and aqueous humor. The cultures were incubated for 24 hours at 37°C, 5 % CO2. In some experiments instead of aqueous humor, the T cells were added to cultures containing the antigen pulsed APC and 30 pg/ml α-MSH. After a 4 hour incubation, TGF-β (5 ng/ml) was added and the cultures were incubated for the remaining 20 hours. The cells were collected and assayed for regulatory activtiy in the adoptive transfer of delayed type hypersensitivity. The culture media was serum-free containing RPMI 1640, 1 mg/ml BSA, 1/500 dilution of ITS+ solution (Collaborative Biomedical Products, Bedford, MA).

T cells (2 x 10⁵ cells) from the inflammatory OVA T cell line cultures were injected along with aqueous humor or α-MSH and TGF-β treated T cells (2x 10⁵ cells) into the tail veins of syngeneic (B10.A) mice in a volume of 200 µl. Within one hour antigen pulsed syngeneic APC (1 x 10⁵ cells) were injected into the right ear pinna of the mouse and ear swelling was measured with a micrometer (Mitsutoyo, Japan) at 24 and 48 hours. Maximum ear swelling occurred at 24 hours and these data are presented as the mean ± SEM of the difference between ear thickness of the APC injected ear and the opposite ear injected with PBS alone, minus their respective original ear thickness. Significance was determined by Student's t test of p = 0.05.

### Primed T Cell In Vitro Assays.

From draining lymph nodes of BALB/c mice immunized 7 days previously with MT-Ag, primed T cells were isolated using CD3 enrichment columns (R&D Systems). T-cells (4 x 10⁵ cells) suspended in serum-free media were added to the wells of a 96 well, round bottom plate (Corning). To the wells were added α-MSH (30 pg/ml) and anti-TCR antibody (2C11; 1 µg/ml) diluted in serum-free culture media. Various concentrations of TGF-β1 or TGF-β2 in media were added. In a second set of experiments TGF-β1 or TGF-β2 at a fixed concentration of 5 ng/ml were added to the wells at various times (0, 2,4 and 6 hours) after addition of anti-TCR antibody. The cultures were incubated for 24 hours and 0.5 µCi of 3H-thymidine (NEM, Boston, MA) was added to the wells and the cultures were incubated for an additional 24 hours. The cells were collected and incorporated radiolabeled was measured by scintillation counting. Production of TGF-β by the treated primed T cells was done by centrifuging the culture plates 24 hours after the addition of anti-TCR antibody, removing the supernatant, washing the cultures once and adding fresh media. The cultures were incubated for an additional 24 hours and the culture supernatant was assayed for TGF-β using the standard CCL-64 bioassay for TGF-β activity as we have previously described.⁸

### Assay For Regulatory T Cell Activities In Vitro.

T cells from peripheral lymph nodes were enriched and cultured as described above except the T cells were treated with α-MSH (30 pg/ml) and anti-TCR. After four hours of incubation TGF-β1 or TGF-β2 at 5 ng/ml was added and the cultures were incubated for an additional 44 hours. The plate was spun down at 250 x g for 10 minutes and all of the supernatant was removed and cells were washed once with media. Freshly isolated primed T cells (4 x 10⁵ cells) were added to all of the wells along with anti-TCR (1 µg/ml) and the cultures were incubated for 48 hours. The culture supernatant was assayed for IFN-γ by sandwich ELISA.

### EXAMPLE II RESULTS

### Aqueous Humor Treated Primed T Cells Suppress DTH.

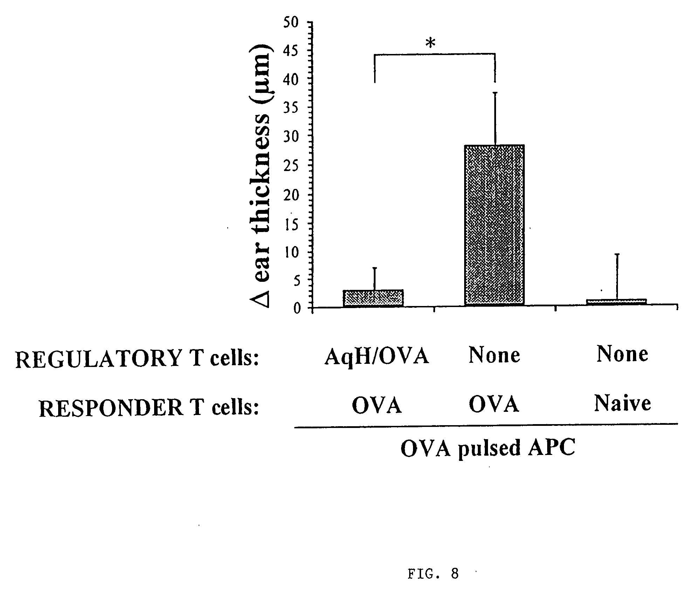

Previously we have demonstrated that primed T cells activated in the presence of aqueous humor, suppress *in vitro* IFN-γ produced by other Th1 cells ⁸. This suggested that these aqueous humor-treated primed T cells should also suppress *in vitro* induction of DTH mediated by Th1 cells. To examine this possibility, aqueous humor-treated T cells, primed to OVA, were injected i.v. along with OVA-reactive Th1 cells. The aqueous humor-treated T cells significantly suppressed the inflammation mediated by the Th1 cells to OVA-pulsed APC that were injected into the pinna of the mouse ear (Figure 8). Therefore, the regulatory T cells induced by aqueous humor suppressed the *in vivo* induction of DTH by other Th1 cells.

Figure 8 is a bar chart showing DTH response in mice as measured by ear swelling, i.e., change in ear thickness (µm), as a function of the type of T cells administered, including whether or not the mice were injected with activated OVA-primed T cells treated with aqueous humor ("Regulatory T cells; AqH/OVA"). Aqueous humor-treated T cells suppress DTH mediated by other Th1 cells. Activated OVA-primed T cells treated with aqueous humor (Regulatory T cells; AqH/OVA) were injected i.v. with DTH mediating T cells (Responder T cells; OVA). OVA-pulsed APC were injected into the ear pinna and ear swelling was measured 24 hours later. The data represent two experiments with similar results and are presented as the percent difference (see methods) in ear thickness ± SEM (n = 5) (P = 0.05).

### Factors Of Aqueous Humor Regulate TCR-Stimulated Proliferation Of Primed T Cells.

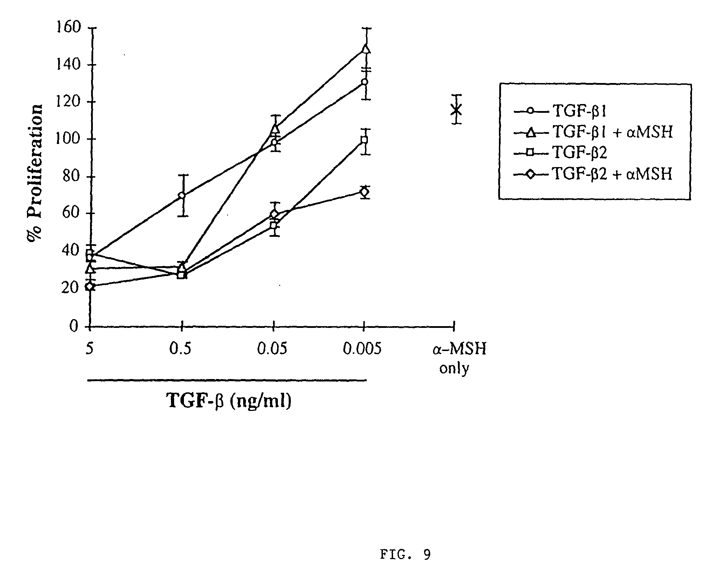

Aqueous humor contains constitutive levels of TGF-β2 and α-MSH^{5,7,9,12}. To examine the effects of TGF-β2 in the presence of α-MSH on TCR-stimulated T cell proliferation, primed T cells were TCR-stimulated in the presence of α-MSH and active TGF-β1 or TGF-β2. Regardless of the presence of α-MSH, increasing concentrations of either TGF-β1 and TGF-β2 suppressed T cell proliferation (Figure 9). It is interesting to find that low concentrations TGF-β1 had either no effect or enhanced T cell proliferation (Figure 9).

Figure 9 is a graph plotting percent proliferation of T cells in response to varying concentrations of TGF-β1 or TGF-β2, with or without α-MSH. There are concentration-dependent effects of TGF-β1 and TGF-β2 on *in vitro* T cell proliferation in the presence of α-MSH. Primed T cells were TCR-stimulated in the presence or absence of 30 pg/ml of α-MSH with either TGF-β1 or TGF-β2 (0.005 - 5.0 ng/ml). Proliferation was measured as counts per minute (CPM) of incorporated ³H-thymidine approximately 48 hours after TCR-stimulation. Data are presented as percent proliferation ± SEM of eight independent experiments. Percent CPM was calculated as the CPM of sample divided by the CPM of untreated TCR-stimulated primed T cells (100% proliferation), minus background.

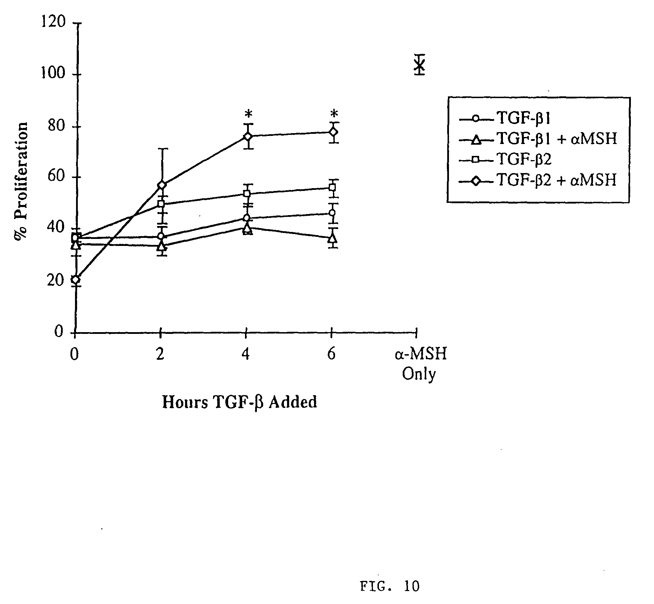

Since only active TGF-β2 can be added to the cultures, it is possible that the proliferative activity observed when the T cells are activated in the presence of whole aqueous humor, occurs because the T cells are influenced in time by increasing levels of latent TGF-β2 being activated in the cultures. This can be simulated by adding active TGF-β2 at various times after TCR-stimulation in the presence of α-MSH. Primed T cells were TCR-stimulated in the presence of α-MSH and, at various times afterwards with active TGF-β1 or TGF-β2. Here not only was it important whether α-MSH was present, but there was also a difference between the effects of TGF-β1 and TGF-β2, as seen in Figure 10.

Figure 10 plots the percentage proliferation as a function of the time at which either TGF-β1 or TGF-β2 was added (Hours TGF-β Added), after TCR-stimulation in the presence of α-MSH. There is a time-dependent effect of TGF-β1 and TGF-β2 on *in vitro* T cell proliferation in the presence of α-MSH. Primed T cells were TCR-stimulated in the presence or absence of 30 pg/ml α-MSH with TGF-β1 or TGF-β2 (5.0 ng/ml) added at different times after TCR-stimulation. Proliferation was measured as counts per minute (CPM) of incorporated 3H-thymidine 48 hours after TCR-stimulation. Data is presented as percent proliferation ± SEM of eight independent experiments as explained in Figure 2. MSH and TGF-β2 treated T cells significantly differed (p = 0.05) from TGF-β2 only treated T cells.

Both TGF-β1 and TGF-β2 added from the start of TCR-stimulation through 6 hours later, suppressed T cell proliferation. However, if α-MSH was present, proliferation was recovered only with the addition of TGF-β2 at or later than 4 hours after TCR-stimulation (Figure 10). Therefore, it is possible that in aqueous humor, the presence of α-MSH antagonizes the antiproliferative activity mediated by activated TGF-β2.

### TGF-β2 Enhances TGF-β Production By Primed T Cells Activated In The Presence Of α-MSH.

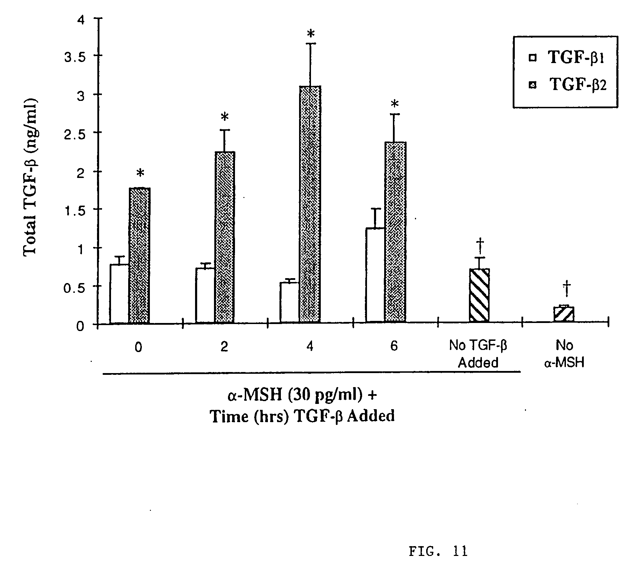

Another characteristic of primed T cells activated in the presence of aqueous humor is that they produce TGF-β.^{A8} Figure 11 shows total TGF-β production in cells treated with α-MSH and either TGF-β1 or TGF-β2, as a function of the time at which the TGF-β was added. There is a time-dependent effect of TGF-β1 and TGF-β2 on TGF-β production by T cells activated in the presence of α-MSH. Primed T cells were TCR-stimulated in the presence of 30 pg/ml α-MSH, with 5.0 ng/ml of TGF-β1 or TGF-β2 added at different times after TCR-stimulation. Culture supernatants were assayed for total TGF-β levels, 48 hours after TCR-stimulation. Data are presented as TGF-β (ng/ml) ± SEM, from eight independent experiments.

TGF-β production was significantly different in primed T cell cultures, where TGF-β1 or TGF-β2 was added, from cultures where no TGF-β of any type was added (p = 0.05).

The primed T cells TCR-stimulated in the presence of α-MSH produced enhanced levels of TGF-β (Figure 11). Addition of TGF-β1 at various times after TCR-stimulation did not change the level of α-MSH-induced TGF-β production (Figure 11). However, the addition of TGF-β2 at various times after TCR-stimulation did enhance α-MSH-induced TGF-β production by the primed T cells (Figure 11). Therefore, the aqueous humor factors α-MSH and TGF-β2 mediate induction of TGF-β-producing T cells, which are potential regulatory T cells.

### The Aqueous Humor Factors α-MSH And TGF-β2 Mediate Induction Of Regulatory T Cells.

Since TGF-β2, when added about 4 hours after TCR-stimulation in the presence of α-MSH, can enhance TGF-β production by the treated T cells, it is possible that α-MSH and TGF-β2 induce activation of regulatory T cells. If regulatory T cells are activated, they should suppress IFN-γ production by other Th1 cells.

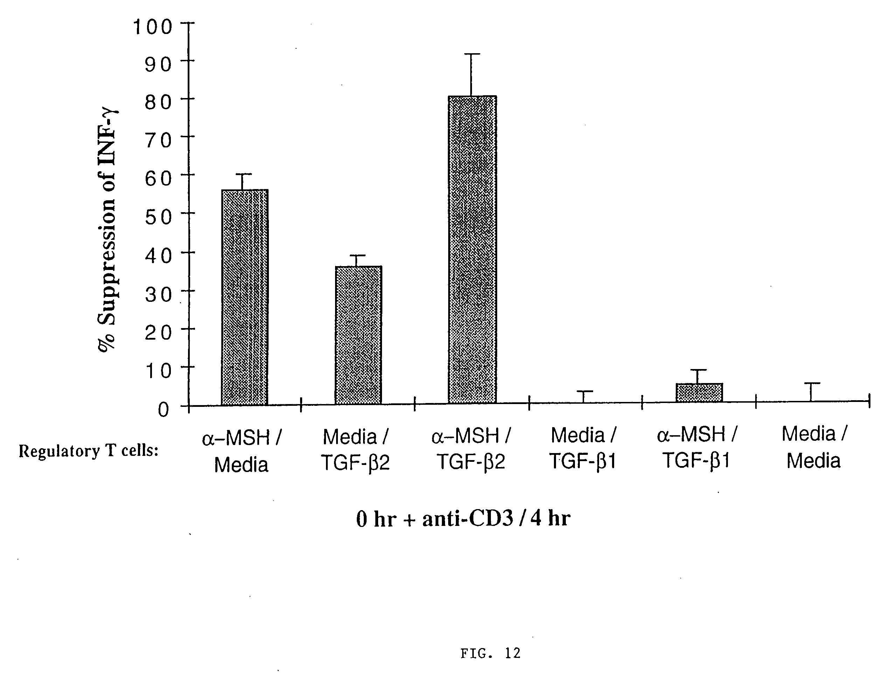

Figure 12 shows the percent suppression in IFN-γ production in activated effector T cells. TGF-β and α-MSH induce regulatory T cell activity. Primed T cells were TCR-stimulated in the presence or absence of 30 pg/ml α-MSH with TGF-β1 or TGF-β2 (5.0 ng/ml) added 4 hours after TCR-stimulation. The treated T cells (Regulatory T cells) were added to cultures of freshly activated primed T cells. Culture supernatants were assayed for IFN-γ 48 hours after addition of treated T cells. Data are presented as percent suppression ± SEM of IFN-γ produced by freshly activated T cells with no added regulatory cells, from eight independent experiments.

Primed T cells TCR-stimulated in the presence of α-MSH and then with TGF-β2 4 hours later, suppressed IFN-γ production by other Th1 cells when the treated primed T cells and Th1 cells were mixed into the same culture (Figure 12). Individually, α-MSH, more than TGF-β2, induced regulatory T cell activity, but the addition of TGF-β2 enhanced the regulatory activity (Figure 12). In contrast, TGF-β1 could not alone or with α-MSH induce regulatory T cells. Moreover, it appears that TGF-β1 antagonized α-MSH-mediated induction of regulatory T cells (Figure 12). The induction of regulatory T cells by α-MSH with TGF-β2 corresponds with the recovered proliferation and enhanced levels of TGF-β production by the treated T cells, as seen in Figures 10 and 11.

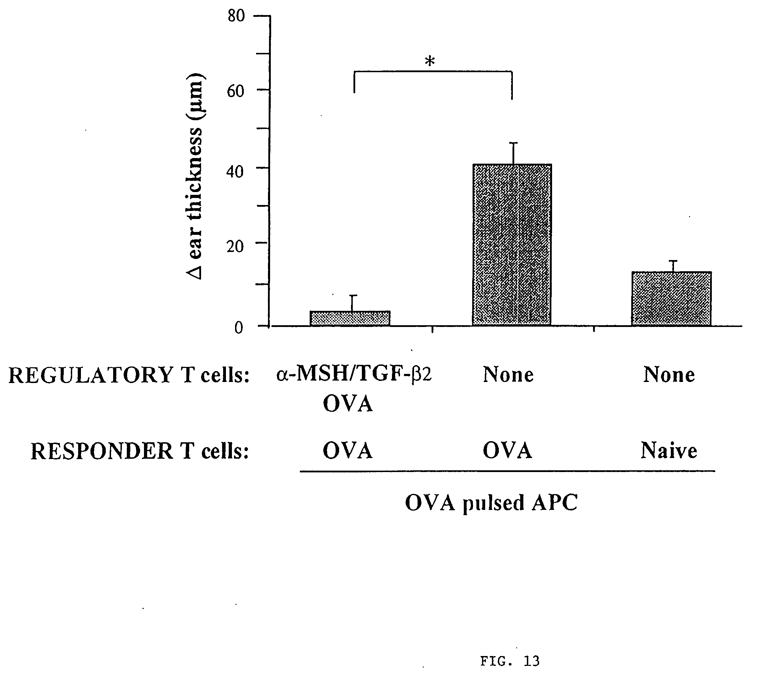

To demonstrate that these factor-induced regulatory T cells could, like aqueous humor-induced regulatory T cells, suppress DTH, primed T cells treated with α-MSH and TGF-β2 were injected i.v. with OVA antigen-primed Th1 cells. The results are shown in Figure 13, a bar chart showing DTH response in mice as measured by ear swelling, i.e., change in ear thickness (µm), as a function of the type(s) of T cells administered, including whether or not the mice were injected with activated, OVA-primed T cells treated with α-MSH and TGF-β2 ("Regulatory T cells; α-MSH / TGF - β2 OVA"). α-MSH/TGF-β2-treated T cells suppress DTH mediated by other, responder T cells (Th1). Activated OVA-primed T cells treated with α-MSH and TGF-β2 (Regulatory T cells; α-MSH/TGF-β2 OVA) were injected i.v. with DTH-mediating T cells (Responder T cells; OVA). OVA-pulsed APC were injected into the ear pinna and ear swelling was measured 24 hours later. The data is representative of two experiments with similar results and is presented as the percent difference in ear thickness ± SEM (n = 5). P = 0.05.

Figure 13 shows that α-MSH - and TGF-β2 - treated, primed T cells suppressed the DTH mediated by the Th1 cells in the pinna of mouse ears injected with OVA pulsed APC. Therefore, α-MSH in conjunction with TGF-β2 mediated activation of functional regulatory T cells, generally known as Th3 cells.

### DISCUSSION OF EXAMPLE II

Aqueous humor and its immunosuppressive factors α-MSH and TGF-β2 mediated induction of Th3 cells. These Th3 cells proliferated and produced TGF-β.^{A8} The induced Th3 cells suppressed Th1 cells from mediating DTH. Our results suggest that the ocular microenvironment has the potential to locally divert primed T cells that are programmed to have a Th1 response, into a Th3 response when activated. The results are also the first report that specific physiologically relevant factors can mediate induction of Th3 cells. The ability for the ocular microenvironment and for α-MSH and TGF-β2 to mediate induction of Th3 cells has implications on the manner by which an immune response is elicited and regulated in the eye.

TGF-β-producing T cells have been described in the oral tolerance models of experimental autoimmune uveitis (EAU) and encephalomyelitis (EAE) ^{A13,A14}. In the oral tolerance models, low doses of orally administered autoantigens induce, through the gut associated lymphoid tissues, (GALT) activation of TGF-β-producing T cells that actively suppress autoimmune disease ^{A15}. These regulatory T cells, also known as Th3 cells, suppress the activity of other disease mediating T cells through their secretion of anti-inflammatory mediators ^{A13- A15}. Such activity results in tolerance to the autoantigen, defined by reduced inflammation at sites of autoantigen-mediated disease. It is possible that the ocular microenvironment, through its constitutive production of α-MSH and TGF-β2, mediates induction of Th3 cells as a potential mechanism to mediate the peripheral tolerance to ocular antigens ^{A16, A17}.

The results indicate that α-MSH is sufficient for induction of regulatory T cells. However, aqueous humor also contains TGF-β2. TGF-β2 itself can also induce activation of regulatory T cells; however, T cell proliferation is relatively suppressed. When TGF-β2 was added to the cultures 4 hours after primed T cells were TCR-stimulated in the presence of α-MSH, there was significant proliferation of, and TGF-β production by, the T cells. Since our primed T cells, when activated, normally function as Th1 cells, the results suggest that α-MSH diverts their Th1 programming into Th3, which diversion is enhanced by TGF-β2. Also, this change requires time to develop within the TCR-stimulated T cells.

In addition, the effects of TGF-β1 and TGF-β2 are different. Regulatory T cell induction does not occur in the presence of TGF-β1. It appears that TGF-β1 clearly suppresses α-MSH induction of regulatory T cell activity. TGF-β1 could be considered under the experimental conditions to be immunosuppressive, while TGF-β2 is immunomodulating. The only possible means that could mediate a differential response to TGF-β1 and TGF-β2 would be through the different receptor requirements for binding the two TGF-β isoforms and the different affinities for TGF-β1 and TGF-β2 by the type II receptor ^{18,19}. Therefore, changes in TGF-β receptor signals, possibly influenced by α-MSH, may mediate the different responses seen by activated primed T cells treated with either TGF-β1 or TGF-β2 in the presence of α-MSH.

The finding that TGF-β2 but not TGF-β1 could induce activation of regulatory T cells, is in line with the finding that it is TGF-β2 protein found in aqueous humor ^{A2, A5, A7}. Under uveitic conditions where the blood-ocular barrier is compromised, entry of TGF-β1 from plasma into the aqueous humor could antagonize α-MSH and TGF-β2 induction of regulatory T cells. This could promote activation, and if active TGF-β1 is at a low concentration, enhance proliferation of activated uveitis-mediating T cells. Recently it has been found that IL-4 and TGF-β can mediate development of TGF-β-producing T cells from a population of naive T cells ^{A11}. Previously we have shown that the effects of α-MSH on primed T cells is similar to the effects of IL-4 on primed T cell activation ^{A10}. Therefore, it is possible that we are observing a similar cytokine-mediated mechanism by aqueous humor in the induction of regulatory T cells with α-MSH inducing IL-4-like signals followed by the effects of TGF-β2 in the T cells. This would be similar to primed/memory T cells entering the ocular microenvironment, being influenced immediately by α-MSH and then, in time, encountering cells producing and activating TGF-β2.

The results here demonstrate that aqueous humor and therefore the ocular microenvironment, possibly through α-MSH and TGF-β2, goes beyond suppressing activation of Th1 cells. ^{A2, A20} Aqueous humor also promotes activation of Th3 cells. Therefore, only specific types of immunological responses are activated within the normal ocular microenvironment. The induction of regulatory, Th3 cells could reinforce the immunosuppressive ocular microenvironment of the eye by their contribution of immunosuppressive lymphokines and by their suppression of Th1 cell activity. The potential for activating autoreactive Th3 cells suggests that their presence *in vivo* could prevent or eliminate clonal expansion and activation of disease-mediating autoimmune Th1 cells.

The ability for the ocular microenvironment to produce constitutive levels of immunosuppressive cytokines that also mediate induction of Th3 cells is an example of how a regional tissue site can manipulate, mold, and coerce an immune response that is tailored for the needs of the tissue. The eye's use of cytokines to regulate the immune response allows for examining the possibility whether these specific immunoregulating factors are neutralized, antagonized, or no longer produced in eyes that are susceptible to or suffering from autoimmune uveitis. It may even be possible to use the same factors to systemically and locally manipulate the immune response to suppress immune-mediated inflammatory diseases. The finding that the ocular microenvironment may induce activation of Th3 cells is an indication that the induction of such regulatory T cells may be a normal physiological occurrence within the eye and that failure of the ocular microenvironment to maintain induction of autoreactive Th3 cells could make it susceptible to autoimmune disease.

### EXAMPLE III

### MATERIALS & METHODS

### Reagents and Animals

The experiments used synthetic α-MSH (Peninsula Laboratories, Belmont, CA); recombinant TGF-β2 and soluble TGF-β receptor type-two (R&D Systems, Minneapolis, MN); the following monoclonal antibodies: anti-CD4 (RM4-4), anti-CD25 (IL-2-receptor-α; 7D4) and anti-CD3ε (145-2C11) (Pharmingen, SanDiego, CA). B10.RIII (Jackson Laboratories Bar harbor, ME) and BALB/c (institute breeding program) female mouse strains 4 to 8-weeks-old were treated with approval by the institutional animal care and use committee in accordance with the US Animal Welfare Act.

### α-MSH treatment of in vivo primed T cells.

*In vivo* primed T cells were obtained from the draining popliteal lymph node of BALB/c mice immunized via a cutaneous foot injection with 0.5 mg desiccated *Mycobacterium tuberculosis* (Difco, Detroit, MI). The T cells were enriched to be about, 99% CD3⁺ by flow cytometry analysis, using a mouse T cell enrichment column (R&D Systems). Into the wells of a 96-well plate were added 100 µl of T cells (4 x 10⁶ cells/ml), 50 µl of diluted α-MSH and 50 µl of anti-CD3ε (1 µg/ml) in *serum-free* culture media. The cultures were incubated for 48 hours and the culture supernatants were assayed using sandwich enzyme linked immunosorbent assays (ELISA) for IFN-γ, IL-4 and IL-10 as described in Example II. Total TGF-β was assayed using the standard CCL-64 bioassay.²⁶ The serum-free culture media⁷ was RPMI 1640 (BioWittaker, Walkerville, MD), 1/75 dilution of sterile 7.5 % BSA solution (Sigma Chemical, St. Louis, MO), 1/500 dilution of 'ITS+' solution (Collaborative Biomedical Products, Bedford, MA). For assaying proliferation the T cell cultures were initially incubated for 24 hours and 20 µl of 50 µCi/ml of ³H-thymidine (NEM, Boston, MA) was added to the wells and the cultures were incubated for an additional 24 hours. The cells were collected onto glass-wool filter paper using a Tomtec Plate Harvester 96 and radiolabel was measured using a Wallac 1205 Betaplate Liquid Scintillation Counter.

### Fluorescence-Activated Cell Sorting.

For immunostaining and flow cytometry, T cells (2 x 10⁶ cells) from 24 hour cultures of the α-MSH-treated activated T cells. The cells were centrifuged and washed once in PBS/BSA buffer (10 mM PBS, 3% BSA). The cells were resuspended in 50 µl of PBS/BSA buffer containing 2 µg of PE-conjugated anti-CD4 and FITC-conjugated anti-CD25 antibodies and incubated for 30 minutes room temperature. The cells were centrifuged, resuspended in 1 ml of PBS/BSA buffer, and washed two times. The stained cells were sorted by a Coulter ELITE cell sorter calibrated for two color fluorescence. The cells were sorted into two populations, CD25⁺/CD4⁺ cells and the remaining cells (CD4⁻ plus CD25⁻CD4⁺ cells). Sorted cells were used immediately in culture experiments.

### Assay For In Vitro Regulatory T Cell Activity.

The α-MSH-treated, TCR-stimulated T cells, as described above, were cultured for 48 hours. The cells were collected and added (2 x 10⁵ cells) to cultures of freshly isolated enriched inflammatory T cells (2 x 10⁵ cells) activated with anti-CD3ε (1 µg/ml). The mixed cell cultures were incubated 48 hours and the culture supernatants were assayed for IFN-γ by sandwich ELISA.

### α-MSH-Treatment of Experimental Autoimmune Uveitis (EAU).

EAU was induced by immunizing B10.RIII mice with 50µg of human interphotoreceptor retinoid binding protein peptide (161-180; IRBPp) emulsified in complete Freund's adjuvant containing 2.0 mg/ml of *Mycobacterium tuberculosis* H37RA.^{B27} The retinal inflammation was clinically assessed every 3 days starting 6 days after the immunization. Some mice received an i.v. injection of 50 µg of α-MSH 10 days and 12 days after the immunization. Funduscopic examinations of the retina were done on eyes topically treated with 0.5% Tropicamide and Neo-Synephirine saline to dilate the pupil. The severity of inflammation was clinically graded on a 0 to 5 scale. No inflammation was scored 0, only white focal lesions of vessels were scored 1, linear lesions of vessels within the half of retina were scored as 2, linear lesions of vessels over half of retina were scored as 3, severe chorioretinal exudates or retinal hemorrhages in addition to the vasculitis were scored as 4, and subretinal hemorrhage or retinal detachments were scored as 5.

### EXAMPLE III RESULTS

### α-MSH suppression of EAU.

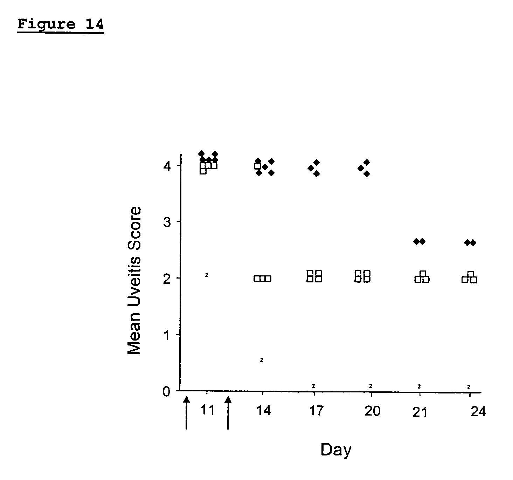

Previously, Lipton and colleagues showed that systemic injections of α-MSH suppressed localized inflammation mediated by innate immune responses induced by IL-1 and endotoxin.^{B15, B17-B19} The results of preceding Example II show that α-MSH suppresses the activation of inflammatory T cells and promotes the activation of TGF-β producing T cells in the ocular microenvironment. Therefore, the experiments of Example III were undertaken to examine whether or not systemic injections of α-MSH could suppress experimental autoimmune uveitis (EAU) in mouse eyes. Mice were immunized to induce EAU as previously described. At the peak of retinal inflammation (Day 11), the experimental mice each received two injections of α-MSH (50 µg/mouse), on days 10 and 12 (Figure 14). Figure 14 shows the mean uveitis score in α-MSH-treated and untreated mice afflicted with experimental autoimmune uveitis (EAU). Suppression of EAU was achieved by intravenous injections of α-MSH. B10.R111 mice were immunized with IRBPp to induce EAU. Five mice were injected with 50 µg of α-MSH i.v. on Days 10 and 12, respectively, (□, Δ) and five mice did not receive α-MSH injections (◆). The data are presented as the mean uveitis score of each mouse.

The mean uveitis score was markedly suppressed in the experimental mice each injected with α-MSH, compared to the untreated, control mice. In one case (Figure 14, Δ), where the uveitis was mild (score 2 on day 11), the injection of α-MSH completely resolved the inflammation (score 0 on day 17). This demonstrates that by elevating the systemic levels of α-MSH, localized T cell-mediated inflammation can be suppressed.

### α-MSH-Regulates T cell Lymphokine Production

Given the findings in Example II that α-MSH mediates induction of TGF-β-producing T cells, and an earlier report that showed that α-MSH suppresses induction of IFN-γ,^{B11} the lymphokine profile of T cells activated in the presence of α-MSH, was examined to see if α-MSH influences production of other lymphokines (Table 1). Enriched, primed T cells were stimulated with anti-CD3ε in the presence of 30 pg/ml of α-MSH, and incubated for 48 hours. The concentration of α-MSH used in these experiments was the mean concentration of α-MSH levels constitutively found in the fluids of aqueous humor and cerebral spinal fluid of mammals.^{B7,B28} Alternatively, α-MSH can be provided in an amount sufficient to give a final concentration of α-MSH of about 30pg/ml *in situ*. The culture supernatants were assayed for IFN-γ, IL-4, IL-10, and TGF-β. α-MSH significantly suppressed IFN-γ production; however, enhanced TGF-β production and had no effect on proliferation (Table 1). In addition, α-MSH suppressed production of IL-10 but not IL-4 by the activated, primed effector T cells (Table 1). This lymphokine profile favored by α-MSH-treated T cells suggests that α-MSH induces activation of regulatory T cells^{B29-B33}, while suppressing inflammatory, effector T cell activity.

**Table 1. Effects of α-MSH on primed T cells activated with anti-CD3ε.**

| Treatment | Cytokine (pg/ml)² | | | | Proliferation (CPM)³ |
|---|---|---|---|---|---|
| | IFN-γ | IL-4 | IL-10 | TGF-β | |
| anti-CD3ε only | 1262 ± 132 | 267 ± 22 | 61 ± 14 | 49 ± 14 | 40171 ± 899 |
| | | | | | |
| anti-CD3ε + α-MSH¹ | 809 ± 44* | 214 ± 63 | 21 ± 9* | 248 ± 73* | 38526 ± 1224 |
| | | | | | |
| - anti-CD3ε | 206 ± 1* | 155 ± 78 | 1 ± 1* | 7 ± 3* | 864 ± 235* |

| | | | | | |
|---|---|---|---|---|---|
| 1. Primed T cells were stimulated with anti-CD3ε in the presence of 30 pg/ml of α-MSH. | | | | | |
| 2. Culture supernatant (48 hours after treatment) were assayed for cytokines by ELISA for IFN-γ, IL-4, IL-10, and by bioassay for TGF-β. Data presented as mean ± SEM of 4 independent experiments each. | | | | | |
| 3. Cell cultures were treated with ³H-thymidine 24 hours after start of assay and incubated for an additional 24 hours before scintillation-counting of the cells. Data presented as counts per minute (CPM) ± SEM of 4 independent experiments. | | | | | |
| * Significantly different (p ≤ 0.05) to primed T cells treated with only anti-CD3ε. | | | | | |

### α-MSH Induces Regulatory T cells

To confirm that α-MSH induces the activation of regulatory T cells, the ability of these T cells to suppress the activity of other, inflammatory T cells was investigated. α-MSH-treated cells were mixed with freshly activated T cells, which normally produce IFN-γ (i.e., are effector T cells primarily of the Th1 type), and assayed the cultures for IFN-γ production (Table 2). The freshly activated T cells were significantly suppressed in their production of IFN-γ by co-culture with unsorted α-MSH-treated T cells (Table 2). This observation corresponds with the Example II findings that α-MSH-treated T cells can suppress DTH. The addition of soluble TGF-β receptor II (sTGF-βRII) to the culture, significantly neutralized the unsorted α-MSH-induced regulatory T cell suppression of IFN-γ production by freshly activated T cells (Table 2). Therefore, the TGF-β produced by α-MSH-induced regulatory T cells is sufficient to suppress IFN-γ production by other, effector T cells (e.g., Th1 cells).

To see if a specific subtype of primed T cells is induced by α-MSH to express regulatory activity, the α-MSH-treated T cells were stained for expression of CD4, a marker for the T helper (Th) cell subset, and the IL-2 receptor-alpha (CD25), a marker of T cell activation. The CD25-expressing CD4 cells (CD25+/CD4+ T cells) were sorted out by fluorescence-activated cell sorting and added to cultures of freshly activated, antigen-primed T cells (i.e., activated without any α-MSH). The immunosuppressive activity was associated with the CD25 expressing CD4 cells and not with other cells in the primed T cell population (Table 2). This finding indicates that α-MSH induces activation of regulatory CD4⁺ T cells that suppress inflammation mediated by other T cells.

**Table 2. Effects of adding α-MSH-treated T cells into cultures of freshly activated, primed effector T cells.**

| Added α-MSH-Treated Cells¹ | IFN-γ (pg/ml)² |
|---|---|
| None | 153 ± 3 |
| Unsorted Cell | 43 ± 7*† |
| Unsorted Cell + sTGF-βRII³ | 130 ± 47† |
| CD25⁺ CD4⁺ cells⁴ | 46 ± 30* |
| CD4⁻ and CD25⁻ CD4⁺ cells⁴ | 175 ± 5 |

| | |
|---|---|
| 1. Cells were added to cultures containing fresh primed T cells and anti-CD3ε. | |
| 2. Supernatants were assayed for IFN-γ 48 hours after addition of α-MSH treated cells. Data presented as mean ± SEM of 4 independent experiments. | |
| 3. Soluble TGF-β receptor type II (sTGF-βRII; 0.5 µg/ml) was added to the culture with the unsorted α-MSH treated T cells. | |
| 4. Primed T cells anti-CD3ε stimulated in the presence of α-MSH for 24 hrs were stained and sorted by fluorescent activated cell sorting. | |
| *Significantly different (p ≤ 0.05) to cultures with no added cells (None). | |
| † Significantly different (p ≤ 0.05). | |

### DISCUSSION OF EXAMPLE III

The preceding results demonstrate that the neuropeptide α-MSH mediates the induction of TGF-β-producing T cells. The lymphokine profile of α-MSH-treated primed T cells is immunosuppressive instead of pro-inflammatory. Therefore, the suppression of autoimmune uveitis appears to be due to α-MSH suppressing production of inflammatory lymphokines by activated autoreactive T cells while inducing activation of regulatory T cells. The Example II results demonstrated that such α-MSH-treated T cells suppress antigen-specific DTH *in vivo* through by-stander suppression.. This observation suggested that α-MSH regulation is mediated through non-antigen specific mechanisms, such as a cytokine. The results shown in Tables 1 and 2 demonstrate that it is at least through the production of TGF-β that α-MSH-treated T cells mediate immunosuppression. Therefore, α-MSH induces activation of regulatory T cells that can mediate regional immunosuppression by producing soluble immunosuppressive factors.

These results also indicate that if the concentration of α-MSH is sufficiently elevated either systemically or regionally, then immunogenic inflammation should be suppressed. Along with mediating the suppression of inflammatory T cells and inducing immunosuppressive lymphokine production, α-MSH also directly antagonizes IL-1, TNF, and IFN-γ-inflammatory activities. In addition, the lymphokines produced by the α-MSH-induced, CD25+ / CD4+ regulatory T cells have the potential to suppress inflammatory macrophage activities.^{B34, B35} Therefore, the suppression of autoimmune disease seen in Figure 14 probably results from the general anti-inflammatory activity of α-MSH (i.e., against innate immunity), along with the effects of α-MSH on activated T cells. There is also the potential that regulatory T cells have been induced by the systemic injections of α-MSH.

The results presented here further suggest that α-MSH has a physiological role in regulating inflammatory immune responses. Its activity within a localized tissue site can regulate the intensity and duration of a T cell-mediated inflammatory response, and possibly, through induction of regulatory T cells, can also affect whether immunogenic inflammation can occur at all (i.e. induce tolerance). In immune-privileged eyes, there is normally a constitutive presence of α-MSH.^{B7} Since the ocular microenvironment has adapted several mechanisms to prevent induction of inflammation, α-MSH may potentially affect immune cells in the eye more so than in other tissue sites. It is likely that within the normal ocular microenvironment, α-MSH mediates induction of TGF-β-producing, CD25+/CD4+ regulatory T cells that in turn mediate peripheral tolerance to ocular autoantigens. Therefore, the ability of α-MSH to selectively regulate the expression of lymphokines in activated T cells means that α-MSH can regulate the induction, intensity, and type of immune response that occurs in a regional tissue site.

The suppression of DTH by adoptive transfer of α-MSH-induced, antigen-primed regulatory T cells, shown in Example II, suggests that such regulatory T cells, if primed by an autoantigen, would suppress induction of inflammatory, T-cell-mediated autoimmune disease. Such autoreactive, regulatory T cells would only be activated in sites where their autoantigen is presented. Through their production of immunosuppressive lymphokines, these CD25+/CD4+, TGF-β-producing T cells would down-regulate or suppress the activation of nearby, autoreactive inflammatory T cells. Such regulation could occur either in the periphery or within a draining lymph node. It is to be seen where α-MSH-induced regulatory T cells migrate *in vivo.*

The present work has demonstrated the importance of the neuropeptide α-MSH in regulating the adaptive immune response, i.e., inflammation mediated by T cells. α-MSH selectively regulates the production of lymphokines by activated effector T cells. This selective immunoregulation by α-MSH has an important role in maintaining immunogenic homeostasis through suppression of inflammation (both innate and T cell-mediated) and possibly through tolerance of autoantigens. It also supports the use of α-MSH's immunosuppressive activities to treat autoimmune diseases.

### USES

As supported by the preceding experimental results and discussions thereof, the invention encompasses a method for generating antigen-specific regulatory T cells that can down-regulate or suppress adaptive immune-mediated inflammation, namely inflammatory responses mediated by activated, primed effector T cells generally of the Th1 subclass. In particular, the method generates regulatory T cells that have a CD4⁺/CD25⁺ phenotype and that produce Transforming Growth Factor β (TGF-β), which suggests that they are Th3-like cells.

In one aspect, the regulatory T cell-generating method comprises exposing CD3-enriched, primed T cells to a specific, presented antigen in the presence of antigen-presenting cells (APC) and the presence of a composition comprising an effective amount of alpha-Melanocyte Stimulating Hormone (α-MSH) or an α-MSH receptor-binding portion thereof. In another aspect, the regulatory T cell- generating method comprises exposing CD3-enriched, primed T cells to a T cell receptor-crosslinking agent in the presence of a composition comprising an effective amount of alpha-Melanocyte Stimulating Hormone (α-MSH) or an α-MSH receptor-binding portion thereof.

The α-MSH receptor-binding portion comprises lysineproline-valine, which represents amino acid residues 11-13 of α-MSH. An effective amount of α-MSH or an α-MSH receptor-binding portion thereof is an amount sufficient to produce an *in situ* concentration of intact α-MSH in the range of about 20-100 pg/ml, preferably about 30 pg/ml or a molar equivalent amount of an α-MSH receptor binding portion of α-MSH, in the immediate vicinity of the primed T cells during the first exposing step.

Either method further comprises, approximately 4-6 hours after the first exposure step has begun (i.e., the exposure of the primed T cells to a T cell activation signal in the presence of α-MSH or binding portion thereof), exposing the primed T cells to an effective amount of TGF-β2. The TGF-β2 enhances the α-MSH's induction of TGF-β-producing, CD4+/CD25+, regulatory T cells. An effective amount of TGF-β2 is an amount sufficient to produce an *in situ* concentration in the range of about 1-10 ng/ml, preferably about 5 ng/ml, in the immediate vicinity of the primed T cells.

The composition comprising α-MSH or a binding portion thereof, may further include TGF-β2 in a timed-release delivery vehicle designed to release the TGF-β2 approximately 4-6 hours after the start of the incubation of the primed T cells with the T cell activation signal (e.g., APC-presented antigen or TCR-crosslinking agent) in the presence of the α-MSH-comprising composition.

The "specific antigen" used in the invention is an antigen recognized by the CD3-enriched, primed T cells, and should be one that is presented to the T cell by an antigen-presenting cell.

In general, primed T cells are understood to be T cells that have previously been exposed to a specific antigen under conditions producing at least one "armed" or "memory" T cell clone or subset that specifically recognizes that antigen and mounts an immune response triggered by engagement of the T cell receptor with that antigen as presented by an antigen-presenting cell. Primed T cells can be derived *in vivo*, by harvesting them from an animal immunized with the specific antigen. Primed T cells can also be produced *in vitro*, by methods well known in the art, such as culturing naive T cells *in vitro* with the specific antigen and with other lymphocytes, antigen presenting cells, cytokines, in culture conditions known to stimulate or generate memory effector T cells that specifically recognize and respond to that antigen. Alternatively, "primed T cells" can be stimulated by crosslinking of the T cell receptors by antibodies (e.g., anti-CD3) or T cell mitogens, such as Concanavalin-A (Con-A), Pokeweed mitogen (PWM), or Phytohemagglutinin (PHA).

Therefore, the regulatory T cell-generating method can also include, prior to exposing primed T cells to α-MSH and/or TGF-β2, stimulating the T cells with an antigen and incubating T cells with an anti-T cell receptor antibody or a T cell mitogen to activate the primed T cells.

Generation of regulatory T cells according to the invention, may be done by culturing primed T cells, the specific antigen, α-MSH (with or without later addition of TGF-β2), and appropriate T-cell culture media *in vitro.* Alternatively, generation of antigen-specific regulatory Th3 cells may be achieved by *in vivo* exposure of primed T cells to the specific antigen in the presence of α-MSH, with addition of TGF-β2 approximately 4-6, preferably about 4, hours after the start of exposure of the primed T cells to the specific antigen and the α-MSH. For instance, a composition comprising both α-MSH and TGF-β2, and the specific antigen to be recognized by the desired TGF-β-producing, CD4+/CD225+, regulatory T cells, may be introduced (e.g., by injection or surgical implantation) into an animal previously immunized with that same specific antigen. (Hence, the immunized animal will have T cells primed to that antigen.) Preferably, the antigen and α-MSH-containing composition are introduced into a localized tissue site (e.g., within an eye, a brain, or a transplant site). Alternatively, antigen-primed T cells may be introduced into the animal along with the specific antigen and a composition comprising α-MSH or α-MSH plus TGF-β2.

Another aspect of the invention encompasses a medicament for down-regulating or suppressing an T cell-mediated inflammation, such as in an autoimmune or a graft rejection response, particularly in a localized tissue site in an animal. Specifically, manufacture of this medicament comprises the following steps conducive to generating regulatory Th3 cells, i.e., CD4⁺ / CD25⁺ T cells that produce TGF-β2:
(a) producing primed T cells by exposing harvested T cells *in vitro* to a specific antigen under conditions enabling stimulation of at least one memory T cell that specifically recognizes said antigen;
(b) exposing the primed T cells *in vitro* to a specific antigen in the presence of a composition comprising alpha-Melanocyte Stimulating Hormone (α-MSH), and in the presence of at least one T cell activating factor, namely a T cell receptor-crosslinking agent (e.g., an anti-TCR antibody or a T cell mitogen) before the T cells generated from step
(c) (which comprise CD4+ CD25+ regulatory T cells) are to be introduced into an aminal.

The medicaments for generating regulatory Th3 cells and of regulating T cell-mediated inflammation, may be used to treat autoimmune disorders, e.g., autoimmune uveitis, in humans and other animals, such as. The medicaments of the invention may also be used in conjunction with transplantation, to suppress or to keep in check, host immune responses responsible for graft rejection.

In all methods and medicaments of the invention, the α-MSH-containing composition comprises α-MSH preferably in a concentration lying within the range of about 30-100 pg/ml. A preferred embodiment of the invention uses α-MSH in a sufficient concentration to provide an *in situ* concentration of at least about 30 pg/ml in the localized tissue site in which generation of regulatory Th3 cells is desired, i.e., in the immediate vicinity of the α-MSH-treated, primed T cells. For instance, in the case of treating a self-contained, small site, e.g., an eye, it may be sufficient to use a composition comprising α-MSH in a concentration of about 30 pg/ml.

When primed T cells are exposed to the specific antigen and a composition comprising both α-MSH and TGF-β2, the TGF-β2 is present in the composition in a timed-release delivery vehicle, preferably in a concentration within the range of about 1-10 ng/ml. More preferably, TGF-β2 is used in a concentration effective to achieve a final concentration of about 5 ng/ml within the local environment of the primed T cells.

Conditions suitable for T cell culture are well-known in the art. For instance, the conditions could include culturing the α-MSH-treated, primed T cells in T cell culture medium, preferably a substantially serum-free one. The treated T cells are typically incubated at about 37°C, for an incubation period within the range of about 18-24 hours, more preferably about 24 hours. Exemplary conditions may be found in preceding Examples I, II, and III.

The invention also encompasses a kit for generating antigen-specific regulatory T cells, thereby regulating T cell-mediated inflammation, comprising: (a) a specific antigen; (b) α-MSH; and (c) an article of manufacture comprising instructions on how to use components (a) and (b) to generate TGF-β-producing, CD4+/CD25+, regulatory T cells. The specific antigen is one to be recognized by the antigen-specific regulatory T cells desired, and for instance, could be a target molecule of an autoimmune disease. The α-MSH or α-MSH receptor-binding-portion thereof is included generally in an amount effective to direct the development primed T cells toward a TGF-β-producing, CD4+/CD25+ phenotype, preferably an amount sufficient to give a final concentration in the range of about 30-100 pg/ml of whole α-MSH or a molar equivalent amount of an α-MSH receptor-binding portion of α-MSH, during exposure of T cells primed to the specific antigen.

The kit further comprises TGF-β2 in an amount effective to enhance the development of the α-MSH-treated primed T cells into TGF-β-producing, CD4+/CD25+, regulatory T cells.

### References

1. Cher, D.J., and T.R. Mosmann. 1987. Two types of murine helper T cell clone. II. delayed-type hypersensitivity is mediated by Th1 clones. *J. Immunol.* 138:3688-3694.
2. VanParijs, L., and A.K. Abbas. 1998. Homeostasis and self-tolerance in the immune system: turning lymphocytes off. *Science* 280:243-248.
3. Taylor, A.W. 1996. Neuroimmuomodulation in immune privilege: role of neuropeptides in ocular immunosuppression. *Neuroimmunomodulation* 3:195-204.
4. Gajewski, T.F., and F.W. Fitch. 1988. Antiproliferative effect of IFN-γ in immune regulation. I. IFN-γ inhibits the proliferation of Th2 but not Th1 murine helper T lymphocyte clones. *J. Immunol.* 140:4245-4252.
5. Gajewski, T.F., J. Joyce, and F.W. Fitch. 1989. Antiproliferative effect of INF-γ in immune regulation. III. Differentail selection of Th1 and TH2 murine helper T lymphocyte clones using recombinant IL-2 and recombinant IFN-γ. *J. Immunol.* 143:15-22.
6. Maggi, E., P. Parronchi, R. Manetti, C. Simonelli, M.-P. Piccinni, F.S. Rugiu, M. De Carli, M. Ricci, and S. Romagnani. 1992. Reciprocal regulatory effects of INF-g and IL-4 on the in vitro development of human Th1 and Th2 clones. *J. Immunol*. 148:2142-2147.
7. Streilein, J.W., B.R. Ksander, and A.W. Taylor. 1997. Immune deviation in relation to ocular immune privilege. *J. Immunol*. 158:3557-60.
8. Constant, S.L., and K. Bottomly. 1997. Induction of TH1 and TH2 CD4⁺ T cell responses. *Annu. Rev. Immunol*. 15:297-322.
9. Taylor, A.W., J.W. Streilein, and S.W. Cousins. 1994. Alpha-melanocyte-stimulating hormone suppresses antigen-stimulated T cell production of gamma-interferon. *Neuroimmunomodulation* 1:188-194.
10. Lee, T.H., A.B. Lerner, and V. Buettner-Janusch. 1961. The isolation and structure of α- and β-melanocyte-stimulating hormones from monkey pituitary glands. *J. Biol. Chem.* 236:1390-1394.
11. Learner, A.B., and M.R. Wright. 1960. *In vitro* frog skin assay for agents that darken and lighten melanocytes. *In* Methods of Biochemical Analysis, vol. 8. D. Glick, editor. Interscience Publishers, Inc., New York, NY. 295-307.
12. Lipton, J.M., and A. Catania. 1997. Anti-inflammatory actions of the neuroimmunomodulator α-MSH. *Immunol. Today* 18:140-145.
13. Shih, S.T., O. Khorram, J.M. Lipton, and S.M. McCann. 1986. Central administration of α-MSH antiserum augments fever in the rabbit. *Amer. J. Physiol*. 250:R803-R806.
14. Holdeman, M., O. Khorram, W.K. Samson, and J.M. Lipton. 1985. Fever-specific changes in central MSH and CRF concentrations. *Amer. J. Physiol.* 248:R125-R129.
15. Martin, L.W., and J.M. Lipton. 1990. Acute phase response to endotoxin: rise in plasma α-MSH and effects of α-MSH injection. *Amer. J. Physiol.* 259:R768-772.
16. Lipton, J.M. 1990. Modulation of host defense by the neuropeptide α-MSH. *Yale J. Bio. Med.* 63:173-182.
17. Star, R.A., N. Rajora, J. Huang, R. Chavez, A. Catania, and J.M. Lipton. 1995. Evidence of autocrine modulation of macrophage nitric oxide synthase by α-MSH. *Proc. Natl. Acad. Sci. USA* 90:8856-8860.
18. Chiao, H., S. Foster, R. Thomas, J. Lipton, and R.A. Star. 1996. α-Melanocyte stimulating hormone reduces endotoxin-induced liver inflammation. *J. Clin. Invest.* 97:2038-2044.
19. Catania, A., N. Rajora, F. Capsoni, F. Minonzio, R.A. Star, and J.M. Lipton. 1996. The neuropeptide α-MSH has specific receptors on neutrophils and reduces chemotaxis in vitro. *Peptides* 17:675-679.
20. Daynes, R.A., B.A. Robertson, B.-H. Cho, D.K. Burnham, and R. Newton. 1987. α-Melanocyte-stimulating hormone exhibits target cell selectivity in its capacity to affect interleukin 1-inducable responses in vivo and in vitro. *J. Immunol*. 139:103-109.
21. Grabbe, S., R.S. Bhardwaj, K. Mahnke, M.M. Simon, T. Schwarz, and T.A. Luger. 1996. α-Melanocyte stimulating hormone induces hapten-specific tolerance in mice. *J. Immunol.* 156:473-478.
22. Bhardwaj, R.S., A. Schwarz, E. Becher, K. Mahnke, Y. Aragane, T. Schwarz, and T.A. Luger. 1996. Pro-opiomelanocortin-derived peptides induce IL-10 production in human monocytes. *J. Immunol*. 156:2517-2521.
23. Taylor, A.W., J.W. Streilein, and S.W. Cousins. 1992. Identification of alpha-melanocyte stimulating hormone as a potential immunosuppressive factor in aqueous humor. *Curr. Eye Res.* 11:1199-1206.
24. Lawrence, D.A. 1991. Identification and activation of latent transforming growth factor β. *Meth. Enzymol.* 198:327-336.
25. Miller, A., O. Lider, A.B. Roberts, M.B. Sporn, and H.L. Weiner. 1992. Suppressor T cells generated by oral tolerization to myelin basic protein suppress both in vitro and in vivo immune responses by the release of transforming growth factor beta after antigen-specific triggering. *Proc. Natl. Acad. Sci. USA* 89:421-5.
26. Chen, Y., V.K. Kuchroo, J.I. Inobe, D.A. Hafler, and H.L. Weiner. 1994. Regulatory T cell clones induced by oral tolerance: suppression of autoimmune encephalomyelitis. *Science* 265:1237-1240.
27. Caspi, R., L. Stiff, R. Morawentz, N. Miller-Rivero, C. Chan, B. Wiggert, R. Nussenblatt, H. Morse, and L. Rizzo. 1996. Cytokine-dependent modulation of oral tolerance in a murine model of autoimmune uveitis. *Ann. N. Y. Acade. Sci.* 778:315-324.
28. Wang, Z.Y., H. Link, A. Ljungdahl, B. Hojeberg, J. Link, B. He, J. Qiao, A. Melms, and T. Olsson. 1994. Induction of interferon-gamma, interleukin-4, and transforming growth factor-beta in rats orally tolerized against experimental autoimmune myasthenia gravis. *Cell. Immun.* 157:353-368.
29. Abehsira-Amar, O., M. Gibert, M. Joliy, J. Thèze, and D.L. Jankovic. 1992. IL-4 plays a dominant role in the differential development of Tho into Th1 and Th2 cells. *J. Immunol.* 148:3820-3829.
30. Weiner, H.L. 1997. Oral tolerance: immune mechanisms and treatment of autoimmune diseases. *Immunol. Today* 18:335-343.
31. Chan, A.C., D.M. Desai, and A. Weiss. 1994. The role of protein tyrosine kinase and protein tyrosine phosphatases in T cell antigen receptor signal transduction. *Annu. Rev. Immunol.* 12:555-592.
32. Alberola-Ila, J., S. Takaki, J.D. Kerner, and R.M. Perlmutter. 1997. Differential signaling by lymphocyte antigen receptors. *Annu. Rev. Immunol.* 15:125-154.
33. Seder, R.A., T. Marth, M.C. Sieve, W. Strober, J.J. Letterio, A.B. Roberts, and B. Kelsall. 1998. Factors involved in the differentiation of TGF-β-producing cells from naive CD4 T cells: IL-4 and IFN-γ have opposing effects, while TGF-β positively regulates its own production. *J. Immunol.* 160:5719-5728.
34. Buggy, J.J. 1998. Binding of α-melanocyte-stimulating hormone to its G-protein-coupled receptor on B-lymphocytes activated the Jak/STAT pathway. *Biochem. J*. 331:211-216.
35. Mountjoy, K.G., L.S. Robbins, M.T. Mortrud, and R.D. Cone. 1992. The cloning of a family of genes that encode the melanocortain receptors. *Science* 257:1248-1251.
36. Kersh, E.N., A.S. Shaw, and P.M. Allen. 1998. Fidelity of T cell activation through multistep T cell receptor ζ phosphorylation. *Science* 281:572-575.
37. Taylor, A.W., and J.W. Streilein. 1996. Inhibition of antigen-stimulated effector T cells by human cerebrospinal fluid. *Neuroimmunomodulation* 3:112-118.
38. Letterio, J.J., and A.B. Roberts. 1998. Regulation of immune responses by TGF-β. *Ann. Rev. Immunol*. 16:137-161.
39. Bennett, N.T., and G.S. Schultz. 1993. Growth factors and wound healing: biochemical properties of growth factors and their receptors. *Amer. J. Surg*. 165:728-737.
40. Holter, W., F.S. Kalthoff, W.F. Pickl, C. Ebner, O. Majdic, D. Kraft, and W. Knapp. 1994. Transforming growth factor-beta inhibits IL-4 and IFN-gamma production by stimulated human T cells. *Int. Immunol.* 6:469-75.
41. Song, X.Y., M. Gu, W.W. Jin, D.M. Klinman, and S.M. Wahl. 1998. Plasmid DNA encoding transforming growth factor-beta1 suppresses chronic disease in a streptococcal cell wall-induced arthritis model. *J. Clin. Invest.* 101:2615-21.
42. Roberts, A.B., and M.B. Sporn. 1988. Transforming growth factor β. *Adv. Can. Res.* 51:107-145.
43. Cromack, D.T., M.B. Sporn, A.B. Roberts, M.J. Merino, L.L. Dart, and J.A. Norton. 1987. Transforming growth factor b levels in rat wound chamber. *J. Surg. Res.* 42:622-628.
44. Wangoo, A., H.T. Cook, G.M. Taylor, and R.J. Shaw. 1995. Enhanced expression of type 1 procollagen and transforming growth factor-beta in tuberculin induced delayed type hypersensitivity. *J Clin Pathol* 48:339-45.
45. Taylor, A.W., P. Alard, D.G. Yee, and J.W. Streilein. 1997. Aqueous humor induces transforming growth factor-beta (TGF-beta)-producing regulatory T-cells. *Curr. Eye Res.* 16:900-8.

A1. Taylor AW. Neuroimmunomodulation in immune privilege: role of neuropeptides in ocular immunosuppression. *Neuroimmunomodulation*. 1996;3:195-204.
A2. Streilein JW, Cousins SW. Aqueous humor factors and their effects on the immune response in the anterior chamber. *Curr Eye Res.* 1990;9:175-182.
A3. Kaiser C, Ksander B, Streilein J. Inhibition of lymphocyte proliferation by aqueous humor. Reg *Immunol.* 1989;2:42-49.
A4. Takeuchi M, Alard P, Streilein JW. TGF-β promotes immune deviation by altering accessory signals of antigen-presenting cells. *J Immunol.* 1998;160:1589-1597.
A5. Granstein R, Staszewski R, Knisely TL, et al. Aqueous humor contains transforming growth factor-β and a small (<3500 daltons) inhibitor of thymocyte proliferation. J *Immunol.* 1990;144:3021-3026.
A6. D'Orazio TJ, Niederkorn JY. A novel role for TGF-β and IL-10 in the induction of immune privilege. *J Immunol.* 1998;160:2089-2098.
A7. Cousins SW, McCabe MM, Danielpour D, Streilein JW. Identification of transforming growth factor-beta as an immunosuppressive factor in aqueous humor. *Investi Ophthalmol Vis Sci.* 1991;32:33-43.
A8. Taylor AW, Alard P, Yee DG, Streilein JW. Aqueous humor induces transforming growth factor-β (TGF-β)-producing regulatory T-cells. *Curr Eye Res.* 1997;16:900-908.
A9. Taylor AW, Streilein JW, Cousins SW. Identification of alpha-melanocyte stimulating hormone as a potential immunosuppressive factor in aqueous humor. *Curr Eye Res.* 1992;11:1199-1206.
A10. Taylor AW, Streilein JW, Cousins SW. Alpha-melanocyte-stimulating hormone suppresses antigen-stimulated T cell production of gamma-interferon. *Neuroimmunomodulation*. 1994;1:188-194.
A11. Seder RA, Marth T, Sieve MC, et al. Factors involved in the differentiation of TGF-β-producing cells from naive CD4⁺ T cells: IL-4 and IFN-γ have opposing effects, while TGF-β positively regulates its own production. *J Immunol.* 1998;160:5719-5728.
A12. Jampel HD, Roche N, Stark WJ, Roberts AB. Transforming growth factor-β in human aqueous humor. *Curr Eye Res.* 1990;9:963-969.
A13. Caspi R, Stiff L, Morawentz R, et al. Cytokine-dependent modulation of oral tolerance in a murine model of autoimmune uveitis. *Ann NY Acade Sci.* 1996;778:315-324.
A14. Chen Y, Kuchroo VK, Inobe JI, Hafler DA, Weiner HL. Regulatory T cell clones induced by oral tolerance: suppression of autoimmune encephalomyelitis. *Science*. 1994;265:1237-1240.
A15. Weiner HL. Oral tolerance: immune mechanisms and treatment of autoimmune diseases. *Immunol Today.* 1997;18:335-343.
A16. Gery I, Steilein JW. Autoimmunity in the eye and its regulation. *Curr Opin Immunol.* 1994;6:938-945.
A17. Streilein J. Peripheral tolerance induction: lessons from immune privileged sites and tissues. *Transp Proc.* 1996;28:2066-2070.
A18. Cheifez S, Weatherbee JA, Tsang ML-S, et al. The transforming growth factor-β system, a complex pattern of cross-reactive ligands and receptors. *Cell.* 1987;48:409-415.
A19. López-Casillas F, Wrana JL, Massagué J. Betaglycan presents ligand to the TGFβ signaling receptor. *Cell.* 1993;73:1435-1444.
A20. Cousins SW, Trattler WB, Streilein JW. Immune privilege and suppression of immunogenic inflammation in the anterior chamber of the eye. *Curr Eye Res.* 1991;10:287-297.
B1. Streilein, J. W. 1999. Immunoregulatory mechanisms of the eye. Prog. Retin. Eye Res. **18**: 357-370.
B4. Cousins, S. W., M. M. McCabe, D. Danielpour & J. W. Streilein. 1991. Identification of transforming growth factor-beta as an immunosuppressive factor in aqueous humor. Investi. Ophthalmol. Vis. Sci. **32**: 33-43.
B5. Granstein, R., R. Staszewski, T. L. Knisely, E. Zeira, R. Nazareno, M. Latina & D. M. Albert. 1990. Aqueous humor contains transforming growth factor-β and a small (<3500 daltons) inhibitor of thymocyte proliferation. J. Immunol. **144:** 3021-3026.
B6. Jampel, H. D., N. Roche, W. J. Stark & A. B. Roberts. 1990. Transforming growth factor-D in human aqueous humor. Curr. Eye Res. **9**: 963-969.
B7. Taylor, A. W., J. W. Streilein & S. W. Cousins. 1992. Identification of alpha-melanocyte stimulating hormone as a potential immunosuppressive factor in aqueous humor. Curr. Eye Res. **11:** 1199-1206.
B8. Taylor, A. W., J. W. Streilein & S. W. Cousins. 1994. Immunoreactive vasoactive intestinal peptide contributes to the immunosuppressive activity of normal aqueous humor. J. Immunol. **153**: 1080-1086.
B10. Ferguson, T. A., S. Fletcher, J. Herndon & T. S. Griffith. 1995. Neuropeptides modulate immune deviation induced via the anterior chamber of the eye. J. Immunol. **155**: 1746-1756.
B11. Taylor, A. W., J. W. Streilein & S. W. Cousins. 1994. Alpha-melanocyte-stimulating hormone suppresses antigen-stimulated T cell production of gamma-interferon. Neuroimmunomodulation **1**: 188-194.
B12. Lee, T. H., A. B. Lerner & V. Buettner-Janusch. 1961. The isolation and structure of α- and β-melanocyte-stimulating hormones from monkey pituitary glands. J. Biol. Chem. **236**: 1390-1394.
B13. Nakanishi, S., A. Inoue, T. Kita, M. Nakamura, A. C. Y. Chang, S. N. Cohen & S. Numa. 1979. Nucleotide sequence of cloned cDNA for bovine corticotropin-b-lipotropin precursor. Nature **278:** 423-427.
B14. Lipton, J. M. 1990. Modulation of host defense by the neuropeptide α-MSH. Yale J. Bio. Med. **63**: 173-182.
B15. Lipton, J. M. & A. Catania. 1997. Anti-inflammatory actions of the neuroimmunomodulator α-MSH. Immunol. Today **18**: 140-145.
B16. Holdeman, M., O. Khorram, W. K. Samson & J. M. Lipton. 1985. Fever-specific changes in central MSH and CRF concentrations. Amer. J. Pysiol. **248**: R125-R129.
B17. Watanabe, T., M. E. Hiltz, A. Catania & J. M. Lipton. 1993. Inhibition of IL-1β-induced periferal inflammation by peripheral and central administration of analogs of the neuropeptide α-MSH. Brain Res. Bull. **32**: 311-314.
B18. Martin, L. W., A. Catania, M. E. Hiltz & J. M. Lipton. 1991. Neuropeptide alpha-MSH antagonizes IL-6- and TNF-induced fever. Peptides **12**: 297-299.
B19. Chiao, H., S. Foster, R. Thomas, J. Lipton & R. A. Star. 1996. α-Melanocyte stimulating hormone reduces endotoxin-induced liver inflammation. J. Clin. Invest. **97**: 2038-2044.
B20. Rajora, N., G. Ceriani, A. Catania, R. A. Star, M. T. Murphy & J. M. Lipton. 1996. α-MSH production, receptors, and influence on neopterin in a human monocyte / macrophage cell line. J Leuk. Biol. **59**: 248-253.
B21. Star, R. A., N. Rajora, J. Huang, R. Chavez, A. Catania & J. M. Lipton. 1995. Evidence of autocrine modulation of macrophage nitric oxide synthase by α-MSH. Proc. Natl. Acad. Sci. USA **90**: 8856-8860.
B22. Catania, A., N. Rajora, F. Capsoni, F. Minonzio, R. A. Star & J. M. Lipton. 1996. The neuropeptide α-MSH has specific receptors on neutrophils and reduces chemotaxis in vitro. Peptides **17**: 675-679.
B23. Chakraborty, A. K., Y. Funasaka, A. Slominski, G. Ermak, J. Hwang, J. M. Pawelek & M. Ichihashi. 1996. Production and release of pro-opiomelanocortin (POMC) derived peptides by human melanocytes and keratinocytes in culture: regulation by ultraviolet B. Biochim. Biophys. Acta **1313**: 130-138.
B24. O'Donohue, T. L. & D. M. Dorsa. 1982. The opiomelanotropinergic neuronal and endocrine systems. Peptides **3**: 353-395.
B26. Taylor, A. W., P. Alard, D. G. Yee & J. W. Streilein. 1997. Aqueous humor induces transforming growth factor-beta (TGF-beta)-producing regulatory T-cells. Curr. Eye Res. **16**: 900-908.
B27. Silver, P. B., L. V. Rizzo, C. C. Chan, L. A. Donoso, B. Wiggert & R. R. Caspi. 1995. Identification of a major pathogenic epitope in the human IRBP molecule recognized by mice of the H-2r haplotype. Investi. Ophthalmol. Vis. Sci. **36**: 946-954.
B28. Taylor, A. W. & J. W. Streilein. 1996. Inhibition of antigen-stimulated effector T cells by human cerebrospinal fluid. Neuroimmunomodulation **3**: 112-118.
B29. Chen, Y., V. K. Kuchroo, J. I. Inobe, D. A. Hafler & H. L. Weiner. 1994. Regulatory T cell clones induced by oral tolerance: suppression of autoimmune encephalomyelitis. Science **265**: 1237-1240.
B30. Caspi, R., L. Stiff, R. Morawentz, N. Miller-Rivero, C. Chan, B. Wiggert, R. Nussenblatt, H. Morse & L. Rizzo. 1996. Cytokine-dependent modulation of oral tolerance in a murine model of autoimmune uveitis. Ann. N.Y. Acade. Sci. **778**: 315-324.
B31. Weiner, H. L. 1997. Oral tolerance: immune mechanisms and treatment of autoimmune diseases. Immunol. Today **18**: 335-343.
B32. Shi, F. D., H. Li, H. Wang, X. Bai, P. H. van der Meide, H. Link & H. G. Ljunggren. 1999. Mechanisms of nasal tolerance induction in experimental autoimmune myasthenia gravis: identification of regulatory cells. J. Immunol. **162**: 5757-5763.
B33. Inobe, J., A. J. Slavin, Y. Komagata, Y. Chen, L. Liu & H. L. Weiner. 1998. IL-4 is a differentiation factor for transforming growth factor-beta secreting Th3 cells and oral administration of IL-4 enhances oral tolerance in experimental allergic encephalomyelitis. Eur. J. Immunol. **28**: 2780-2790.
B34. Takeuchi, M., M. M. Kosiewicz, P. Alard & J. W. Streilein. 1997. On the mechanisms by which transforming growth factor-β2 alters antigen-presenting abilities of macrophages on T cell activation. Eur. J. Immunol. **27**: 1648-1656.
B35. Tsunawaki, S., M. Sporn, A. Ding & C. Nathan. 1988. Deactivation of macrophages by transforming growth factor-β. Nature **334:** 260-262.

## Claims

1. A method for generating antigen-specific, regulatory CD4+/CD25+ T cells that produce Transforming Growth Factor β (TGF-β), comprising the steps of:
exposing CD3-enriched, primed T cells to a specific antigen in the presence of antigen-presenting cells or to a T cell receptor (TCR)-crosslinking agent in the presence of a composition comprising an effective amount of alpha-Melanocyte Stimulating Hormone (α-MSH) or an α-MSH receptor-binding portion of α-MSH, wherein the specific antigen is an antigen recognized by the primed T cells wherein the exposing step is performed in vitro under T cell culture conditions;
approximately 4-6 hours after said first exposure step has begun, additionally exposing the primed T cells to an effective amount of Transforming Growth Factor-β2 (TGF-β2).

2. The method of claim 1, wherein the exposure to TGF-β2 is achieved by including in the composition, an effective amount of TGF-β2 in an timed-release delivery vehicle.

3. The method of one of the preceding claims; comprising the steps of:
(a) inducing TGF-β-producing, regulatory T cells by exposing T cells which have been harvested from an animal in vitro to a specific antigen under culture conditions enabling stimulation of at least one primed memory T cell that specifically recognizes said antigen;
(b) exposing the primed T cells in vitro to a specific antigen in the presence of a composition comprising an effective amount of alpha-Melanocyte Stimulating Hormone (α-MSH) or an α-MSH receptor-binding portion of α-MSH, and in the presence of at least one T cell receptor (TCR)-crosslinking agent, under T cell culture conditions, before the primed T cells are injectable into an animal;
wherein step (b) further comprises the addition of an effective amount of TGF-β2, approximately 4-6 hours after the start of the exposure of the primed T cells to the specific antigen and the α-MSH.

4. The method of claim 3, wherein, after step (b), the primed T cells treated in accordance with step (b) are enriched for CD4+/CD25+, TGF-β-producing T cells.

5. The method of one of the preceding claims, wherein the TCR-crosslinking agent is an anti-CD3 monoclonal antibody.

6. The method of one of the claims 1 to 4, wherein the TCR-crosslinking agent is a T cell mitogen selected from the group consisting of: concanavalin-A (ConA); phytohemagglutinin (PHA); and pokeweed mitogen (PWM).

7. The method of one of the preceding claims, wherein the effective amount of α-MSH or of an α-MSH receptor-binding portion thereof, is an amount sufficient to produce an in situ concentration of at least approximately 30 pg/ml of whole α-MSH or a molar equivalent amount of an α-MSH receptor-binding portion of α-MSH, in the immediate vicinity of the primed T cells during the exposing step.

8. The method of claim 7, wherein the effective amount of α-MSH or α-MSH receptor-binding portion thereof, is an amount sufficient to produce an in situ concentration in the range of approximately 30-100 pg/ml in the immediate vicinity of the primed T cells during the exposing step.

9. The method of one of the preceding claims, wherein the effective amount of TGF-β2 is an amount sufficient to produce an in situ TGF-β2 concentration that lies within the range of approximately 1-10 ng/ml in the immediate vicinity of the primed T cells during the exposing step.

10. The method of claim 9, wherein the effective amount of TGF-β2 is an amount sufficient to produce an in situ TGF-β2 concentration of approximately 5.0 ng/ml in the immediate vicinity of the primed T cells during the exposing step.

11. The method of one of the preceding claims, wherein the α-MSH receptor-binding portion thereof comprises lysineproline-valine.

12. The method of one of the preceding claims, wherein the exposing step comprises incubating the T cells in vitro with the specific antigen and the composition at approximately 37°C, for a period within the range of approximately 18-24 hours, in substantially serum-free T cell culture conditions.

13. The method of claim 12, wherein the substantially serum-free T cell medium includes RPMI 1640, an approximately 500-fold dilution of ITS+ solution and approximately 0.1 % bovine serum albumin.

14. The method of claim 3, wherein the animal is a human, a mouse, a rat, a dog, a cat, a rabbit, or a horse.

15. Use of the method of claims 1 to 14 for preparing a drug, in particular a drug for down-regulating an autoimmune response or a graft rejection response or an other T cell-mediated inflammatory response.

16. A kit for generating antigen-specific, regulatory CD4+/CD25+ T cells that produce Transforming Growth Factor β (TGF-β), comprising:
(a) a specific antigen;
(b) α-MSH ;
(c) an article of manufacture comprising instructions on how to use components (a) and (b) to generate TGF-β-producing, CD4+/CD25+, regulatory T cells; and
(d) TGF-β2, and wherein the article of manufacture further comprises instructions for using the TGF-β2.

17. The kit of claim 16, wherein the specific antigen comprises a target molecule of an autoimmune disorder.

18. The kit of claim 17, wherein the target molecule is a member selected from the group consisting of: a glycoprotein; a protein; a polypeptide; a synthetic amino acid polypeptide; a recombinant amino acid polypeptide; a carbohydrate moiety; an oligonucleotide; a DNA; a RNA; and a whole tnicroorganism.

19. The kit of one of the claims 16 to 18 for medical use.

20. Use of the kit of one of the claims 16 to 18 for preparing a drug for down-regulating an autoimmune response or a graft rejection response or an other T cell-mediated inflammatory response.

## Patentansprüche

1. Verfahren zum Erzeugen von antigenspezifischen, regulatorischen CD4+/CD25+-T-Zellen, die den Transformationswachstumsfaktor β (TGF-β) erzeugen, mit den Schritten:
Aussetzen von mit CD3 angereicherten, Primer-T-Zellen einem spezifischen Antigen in Gegenwart von Antigen präsentierenden Zellen oder einem T-Zellen-Rezeptor- (TCR) Vernetzungsmittel in Gegenwart einer Zusammensetzung mit einer wirksamen Menge von Alpha-Melanozyten stimulierendem Hormon (α-MSH) oder eines α-MSH-Rezeptorbindungsteils von α-MSH, wobei das spezifische Antigen ein Antigen ist, das von den Primer-T-Zellen erkannt wird, wobei der Aussetzungsschritt in vitro unter T-Zellkulturbedingungen durchgeführt wird;
ungefähr 4-6 Stunden, nachdem der erste Aussetzungsschritt begonnen hat, zusätzliches Aussetzen der Primer-T-Zellen einer wirksamen Menge des Transformationswachstumsfaktors β2 (TGF-β2).

2. Verfahren nach Anspruch 1, wobei das Aussetzen dem TGF-β2 erreicht wird, indem in die Zusammensetzung eine wirksame Menge von TGF-β2 in einem Abgabeträger mit zeitlich gesteuerter Freisetzung eingeschlossen wird.

3. Verfahren nach einem der vorangehenden Ansprüche mit den Schritten:
(a) Induzieren von TGF-β erzeugenden, regulatorischen T-Zellen, indem T-Zellen, die von einem Tier geerntet wurden, in vitro einem spezifischen Antigen unter Kulturbedingungen ausgesetzt werden, die die Stimulation von mindestens einer Primer-Gedächtnis-T-Zelle ermöglichen, die das Antigen spezifisch erkennt;
(b) Aussetzen der Primer-T-Zellen in vitro einem spezifischen Antigen in Gegenwart einer Zusammensetzung mit einer wirksamen Menge eines Alpha-Melanozyten stimulierenden Hormons (α-MSH) oder eines α-MSH-Rezeptorbindungsteils von α-MSH und in Gegenwart von mindestens einem T-Zellenrezeptor- (TCR) Vernetzungsmittel unter T-Zellenkulturbedingungen, bevor die Primer-T-Zellen in ein Tier injizierbar sind;
wobei Schritt (b) ferner die Zugabe einer wirksamen Menge von TGF-β2 ungefähr 4-6 Stunden nach dem Beginn der Aussetzung der Primer-T-Zellen dem spezifischen Antigen und dem α-MSH umfasst.

4. Verfahren nach Anspruch 3, wobei nach Schritt (b) die Primer-T-Zellen, die gemäß Schritt (b) behandelt wurden, mit TGF-β erzeugenden CD4+/CD25+-T-Zellen angereichert werden.

5. Verfahren nach einem der vorangehenden Ansprüche, wobei das TCR-Vernetzungsmittel ein monoklonaler Anti-CD3-Antikörper ist.

6. Verfahren nach einem der Ansprüche 1 bis 4, wobei das TCR-Vernetzungsmittel ein T-Zellen-Mitogen ist, das aus der Gruppe ausgewählt ist, die aus folgendem besteht: Concanavalin A (ConA); Phytohemagglutinin (PHA); und Kermesbeeren-Mitogen (PWM).

7. Verfahren nach einem der vorangehenden Ansprüche, wobei die wirksame Menge von α-MSH oder eines α-MSH-Rezeptorbindungsteils davon eine Menge ist, die ausreicht, um eine In-situ-Konzentration von mindestens ungefähr 30 pg/ml von ganzem α-MSH oder einer Moläquivalentmenge eines α-MSH-Rezeptorbindungsteils von α-MSH in der unmittelbaren Nähe der Primer-T-Zellen während des Aussetzungsschritts zu erzeugen.

8. Verfahren nach Anspruch 7, wobei die wirksame Menge von α-MSH oder eines α-MSH-Rezeptorbindungsteils davon eine Menge ist, die ausreicht, um eine In-situ-Konzentration im Bereich von ungefähr 30-100 pg/ml in der unmittelbaren Nähe der Primer-T-Zellen während des Aussetzungsschritts zu erzeugen.

9. Verfahren nach einem der vorangehenden Ansprüche, wobei die wirksame Menge von TGF-β2 eine Menge ist, die ausreicht, um eine In-situ-TGF-β2-Konzentration, die innerhalb des Bereichs von ungefähr 1-10 ng/ml liegt, in der unmittelbaren Nähe der Primer-T-Zellen während des Aussetzungsschritts zu erzeugen.

10. Verfahren nach Anspruch 9, wobei die wirksame Menge von TGF-β2 eine Menge ist, die ausreicht, um eine In-situ-TGF-β2-Konzentration von ungefähr 5,0 ng/ml in der unmittelbaren Nähe der Primer-T-Zellen während des Aussetzungsschritts zu erzeugen.

11. Verfahren nach einem der vorangehenden Ansprüche, wobei der α-MSH-Rezeptorbindungsteil davon Lysinprolin-valin umfasst.

12. Verfahren nach einem der vorangehenden Ansprüche, wobei der Aussetzungsschritt das Inkubieren der T-Zellen in vitro mit dem spezifischen Antigen und der Zusammensetzung bei ungefähr 37°C für einen Zeitraum innerhalb des Bereichs von ungefähr 18-24 Stunden unter im Wesentlichen serumfreien T-Zellenkulturbedingungen umfasst.

13. Verfahren nach Anspruch 12, wobei das im Wesentlichen serumfreie T-Zellenmedium RPMI 1640, eine ungefähr 500-fache Verdünnung einer ITS+-Lösung und ungefähr 0,1 % Rinderserumalbumin umfasst.

14. Verfahren nach Anspruch 3, wobei das Tier ein Mensch, eine Maus, eine Ratte, ein Hund, eine Katze, ein Kaninchen oder ein Pferd ist.

15. Verwendung des Verfahrens nach den Ansprüchen 1 bis 14 zur Herstellung eines Arzneimittels, insbesondere eines Arzneimittels für die Herabregulierung einer Autoimmunreaktion oder einer Transplantatabstoßreaktion oder einer anderen durch T-Zellen vermittelten Entzündungsreaktion.

16. Kit zum Erzeugen von antigenspezifischen, regulatorischen CD4+/CD25+-T-Zellen, die den Transformationswachstumsfaktor β (TGF-β) erzeugen, mit:
(a) einem spezifischen Antigen;
(b) α-MSH;
(c) einem Herstellungsgegenstand mit Anweisungen dafür, wie die Komponenten (a) und (b) zu verwenden sind, um TGF-β erzeugende, regulatorische CD4+/CD25+-T-Zellen zu erzeugen; und
(d) TGF-β2, und wobei der Herstellungsgegenstand ferner Anweisungen zur Verwendung des TGF-β2 umfasst.

17. Kit nach Anspruch 16, wobei das spezifische Antigen ein Zielmolekül einer Autoimmunstörung umfasst.

18. Kit nach Anspruch 17, wobei das Zielmolekül ein Element ist, das aus der Gruppe ausgewählt ist, die aus folgendem besteht: einem Glycoprotein; einem Protein; einem Polypeptid; einem synthetischen Aminosäurepolypeptid; einem rekombinanten Aminosäurepolypeptid; einem Kohlenhydratanteil; einem Oligonukleotid; einer DNA; einer RNA; und einem ganzen Mikroorganismus.

19. Kit nach einem der Ansprüche 16 bis 18 zur medizinischen Verwendung.

20. Verwendung des Kits nach einem der Ansprüche 16 bis 18 zur Herstellung eines Arzneimittels zum Herabregulieren einer Autoimmunreaktion oder einer Transplantatabstoßreaktion oder einer anderen durch T-Zellen vermittelten Entzündungsreaktion.

## Revendications

1. Méthode pour générer des cellules T régulatrices CD4+/CD25+ spécifiques d'un antigène qui produisent un Facteur de Croissance Transformant de type β (TGF-β), comprenant les étapes de :
exposer des cellules T stimulées, enrichies en CD3, à un antigène spécifique en présence de cellules comportant l'antigène ou un agent de réticulation de récepteur de cellule T (TCR) en présence d'une composition comprenant une quantité active de α-MSH (alpha-mélanostimuline) ou d'un fragment de α-MSH se liant au récepteur de α-MSH, où l'antigène spécifique est un antigène reconnu par les cellules T stimulées, où l'étape d'exposition est réalisée *in vitro* dans des conditions de culture de cellules T ;
environ 4 à 6 heures après que la première étape d'exposition a commencé, exposer en plus les cellules T stimulées à une quantité efficace de Facteur de Croissance Transformant β2 (TGF-β2).

2. Méthode selon la revendication 1, dans laquelle l'exposition au TGF-β2 est réalisée en incluant dans la composition une quantité active de TGF-β2 dans un vecteur permettant son relargage progressif.

3. Méthode selon l'une quelconque des revendications précédentes, comprenant les étapes de :
(a) induire des cellules T régulatrices, produisant du TGF-β, en exposant *in vitro* des cellules T récoltées sur un animal à un antigène spécifique dans des conditions de culture permettant la stimulation d'au moins une cellule T mémoire stimulée qui reconnaît spécifiquement ledit antigène ;
(b) exposer les cellules T stimulées *in vitro* à un antigène spécifique en présence d'une composition comprenant une quantité active d'alpha-mélanostimuline (α-MSH) ou d'une partie de α-MSH se liant au récepteur de α-MSH, et en présence d'au moins un agent de réticulation de récepteur de cellule T (TCR), dans des conditions de culture de cellules T, avant que les cellules T stimulées soient injectables dans un animal ;
où l'étape (b) comprend en outre l'addition d'une quantité efficace de TGF-β2, environ 4 à 6 heures après le début de l'exposition des cellules T stimulées à l'antigène spécifique et à la α-MSH.

4. Méthode selon la revendication 3, où, après l'étape (b), les cellules T stimulées traitées en accord avec l'étape (b) sont enrichies en cellules T CD4+/CD25+ produisant du TGF-β.

5. Méthode selon l'une quelconque des revendications précédentes, dans laquelle l'agent de réticulation de TCR est un anticorps monoclonal anti-CD3.

6. Méthode selon l'une quelconque des revendications 1 à 4, dans laquelle l'agent de réticulation de TCR est un agent mitogène de cellule T sélectionné dans le groupe constitué de : concanavaline A (ConA) ; phytohémaglutinine (PHA) ; et mitogène phytolaque (pokeweed mitogen, PWM).

7. Méthode selon l'une quelconque des revendications précédentes, dans laquelle la quantité active de α-MSH ou d'une partie de celui-ci se combinant au récepteur de α-MSH est une quantité suffisante pour produire, pendant l'étape d'exposition, une concentration *in situ* d'au moins environ 30 pg/mL d'un α-MSH complet ou une quantité équivalente molaire d'une partie d'un α-MSH se combinant au récepteur de α-MSH, à proximité immédiate des cellules T stimulées.

8. Méthode selon la revendication 7, dans laquelle la quantité active de α-MSH ou d'une partie de celui-ci se liant au récepteur de α-MSH est une quantité suffisante pour produire, pendant l'étape d'exposition, une concentration *in situ* de l'ordre d'environ 30 à 100 pg/mL à proximité immédiate des cellules T stimulées.

9. Méthode selon l'une quelconque des revendications précédentes, dans laquelle la quantité active de TGF-β2 est une quantité suffisante pour produire, pendant l'étape d'exposition, une concentration *in situ* de TGF-β2 de l'ordre d'environ 1 à 10 ng/mL à proximité immédiate des cellules T stimulées.

10. Méthode selon la revendication 9, dans laquelle la quantité efficace de TGF-β2 est une quantité suffisante pour produire, pendant l'étape d'exposition, une concentration *in situ* de TGF-β2 d'environ 5,0 ng/mL à proximité immédiate des cellules T stimulées.

11. Méthode selon l'une quelconque des revendications précédentes, dans laquelle la partie de α-MSH se liant au récepteur de celui-ci comprend la séquence lysine - proline - valine.

12. Méthode selon l'une quelconque des revendications précédentes, dans laquelle l'étape d'exposition comprend l'incubation *in vitro* des cellules T avec l'antigène spécifique et la composition à environ 37 °C pendant une période de l'ordre de 18 à 24 heures environ, dans des conditions de culture de cellules T en l'absence substantielle de sérum.

13. Méthode selon la revendication 12, dans laquelle le milieu de culture de cellules T sans sérum inclut le RPMI 1640, une solution d'ITS+ diluée 500 fois environ et environ 0,1 % d'albumine de sérum bovin.

14. Méthode selon la revendication 3, dans laquelle l'animal est un être humain, une souris, un rat, un chien, un chat, un lapin ou un cheval.

15. Utilisation de la méthode selon les revendications 1 à 14 pour préparer un médicament, en particulier un médicament pour réguler à la baisse une réaction auto-immunitaire, une réaction de rejet de greffe ou une autre réaction inflammatoire contrôlée par les cellules T.

16. Kit pour générer des cellules T régulatrices CD4+/CD25+, spécifiques d'un antigène qui produisent un Facteur de Croissance Transformant de type β (TGF-β), comprenant :
(a) un antigène spécifique ;
(b) de l'α-MSH ;
(c) un objet fabriqué comprenant des instructions sur la manière d'utiliser les composants (a) et (b) pour générer des cellules T régulatrices CD4+/CD25+, produisant du TGF-β ; et
(d) du TGF-β2, et où l'objet fabriqué comprend en outre des instructions sur la manière d'utiliser le TGF-β2.

17. Kit selon la revendication 16, dans lequel l'antigène spécifique comprend une molécule cible d'un trouble auto-immun.

18. Kit selon la revendication 17, dans lequel la molécule cible est un membre du groupe consistant en : une glycoprotéine ; une protéine ; un polypeptide ; un polypeptide synthétique ; un polypeptide recombinant ; un composé de type hydrate de carbone ; un oligonucléotide ; un ADN ; un ARN ; et un micro-organisme complet.

19. Kit selon l'une quelconque des revendications 16 à 18 pour usage médical.

20. Utilisation du kit selon l'une quelconque des revendications 16 à 18 pour préparer un médicament permettant de réguler à la baisse une réaction auto-immunitaire, une réaction de rejet de greffe ou une autre réaction inflammatoire contrôlée par les cellules T.
